Europäisches Patentamt

European Patent Office

Office européen des brevets

(19)

(11) Publication number: **0 253 241**

**A1**

(12)

# EUROPEAN PATENT APPLICATION

(21) Application number: 87109628.5

(22) Date of filing: 03.07.87

(51) Int. Cl.⁴ **C12N 15/00** , C12N 9/72 ,
C07H 21/04 , C12N 5/00

The applicant has filed a statement in accordance with Rule 28 (4) EPC (issue of a sample only to an expert). Accession number(s) of the deposit(s): IFO 14636, 14637, 14638, ATCC CCL61.

(30) Priority: 03.07.86 JP 156936/86
18.02.87 JP 36495/87

(43) Date of publication of application:
20.01.88 Bulletin 88/03

(84) Designated Contracting States:
BE CH DE ES FR GB LI NL SE

(71) Applicant: GREEN CROSS CORPORATION
15-1, Imabashi 1-chome Higashi-ku
Osaka-shi
Osaka(JP)

(72) Inventor: Kasai, Shunji
Fleur Fujisaka 2-203 50,Fujisaka Motomachi
2-chome
Hirakata-shi Osaka(JP)
Inventor: Hiramatsu, Ryuji
14-1-1008, Kayashima Shinwa-cho
Neyagawa-shi Osaka(JP)
Inventor: Uno, Shusei
24-9, Kita Nakafuri 1-chome
Kirakata-shi Osaka(JP)
Inventor: Nagai, Masanori
10-10, Nishiyama Waki
Yahata-shi Kyoto(JP)
Inventor: Arimura, Hirofumi
2-18-1-401, Uenozaka
Toyonaka-shi Osaka(JP)

(74) Representative: Hansen, Bernd, Dr.rer.nat. et al
Hoffmann, Eitle & Partner Patentanwälte
Arabellastrasse 4
D-8000 München 81(DE)

(54) Human prourokinase mutants, method for producing the same, DNA sequences encoding the same, plasmids containing the same and transformants containing the same.

(57) A human prourokinase mutant is disclosed, in which the entire or a partial epidermal growth factor domain of human prourokinase is deleted or is replaced by one or more different amino acid residues.

# HUMAN PROUROKINASE MUTANTS, METHOD FOR PRODUCING THE SAME, DNA SEQUENCES ENCODING THE SAME, PLASMIDS CONTAINING THE SAME AND TRANSFORMANTS CONTAINING THE SAME

## FIELD OF THE INVENTION

This invention relates to mutants or variants of human prourokinase derived from human prourokinase (hereinafter referred to as "human PUK") by modification of its molecular structure. a method of producing the same, DNA sequences coding for the human PUK mutants, plasmids containing the DNA sequences inserted therein, and transformants containing the plasmids. More particularly, the invention relates to methods for providing a human PUK mutant by deleting an entire or a partial specific gene region or substituting an entire or a partial specific gene region with one or more different nucleic acid residues, followed by expressing the gene using recombinant DNA techniques.

## BACKGROUND OF THE INVENTION

Urokinase is a well-known fibrinolytic enzyme. This enzyme has been purified from human urine and human kidney cell culture fluids. Recently, it has also become possible to produce urokinase using recombinant DNA technology (Japanese Patent Application (OPI) No. 60-I8059I corresponding to EP-0I54272A and U.S. Application Serial No. 703,678 filed February I5, I985). However. the thus obtained urokinase has a drawback in that when it is used in large doses. degradation and activation of coagulation and fibrinolysis factors occurs which leads to bleeding. On the other hand, it has been found that an inactive-form (precursor) of human urokinase, namely human PUK, produced by human kidney cells (Japanese Patent Application (OPI) No. 60-6298I (corresponding to EP-0I39447A and U.S. Application Serial No. 648,I34 filed September 7, I984); J. Biol. Chem., 260, I2377 (I985), unlike urokinase, dissolves thrombi without inducing any substantial bleeding (Cell Struc. Func., I0, I5I (I985)).

Human PUK is composed of three functional domains, i.e., (I) the epidermal growth factor (hereinafter abbreviated as "EGF") domain, the (2) kringle domain and (3) the enzyme activity domain (Hoppe-Seyler's, Z., Physiol. Chem., 363, II55 (I982)).

However, this substance, like urokinase, has a drawback in that it has a short half-life in blood. As a result, relatively large doses are required for the purpose of thrombolysis.

## SUMMARY OF THE INVENTION

Accordingly, it is an object of the present invention to provide fibrinolytic enzymes having a longer half-life in blood as compared with human PUK and, like human PUK, induces only a very low level of bleeding a method of producing the same, DNA sequences coding for the fibrinolytic enzymes, plasmids containing the DNA sequences inserted therein, the transformants containing the plasmids.

The above object of the present invention has been met by recombinant human PUK mutants in which the entire or a partial EGF domain of human PUK is deleted or substituted by one or more different amino acid residues and has a longer half-life in blood than that of human PUK but like human PUK, induces only a very low level of bleeding DNAs coding for the human PUK mutants, plasmids containing the DNA sequences inserted therein, hosts containing the plasmids and methods of producing the human PUK mutants.

## BRIEF DESCRIPTION OF THE DRAWINGS

Fig. I shows the construction scheme for the human PUK expression vector, pSV-UKI0, which has a new restriction enzyme site;

Fig. 2 shows the restriction enzyme map of the human PUK expression, vector, pSV-G₁-preUK; ·

Fig. 3 shows the structure, in the vicinity of the preUK cDNA, of pSV-G₁-preUK;

Fig. 4 shows the insertion of a preUK cDNA fragment into the MI3mpI8R plasmid;

Fig. 5 shows the construction scheme for an open circular plasmid, MI3-UKI-OC;

Fig. 6 shows a process for introducing a Sac I site and an NdeI site into the human PUK gene;

Fig. 7 shows the structure of the mutant PUK expression vector, pSV-UKII;

Fig. 8 show the restriction enzyme map of the plamid, pSV-UK50;

Fig. 9 shows the process for preparing plasmids pUH7 to 9;

Fig. I0 shows an expression vector for use in Escherichia coli;

Fig. II shows an expression vector for use in Bacillus subtilis; and

Fig. I2 shows an expression vector for use in Saccharomyces cerevisiae.

## DETAILED DESCRIPTION OF THE INVENTION

It has been reported that among the 4II amino acid residues containing human PUK, 49 amino acid residues from the N-terminal serine to the 49th amino acid residue threonine constitute the EGF domain (Riccio, A. et al., Nucleic Acids Res., I3, 2759-277I (I985)). Therefore, the present invention provides a method for producing human PUK mutants in which the entire or a partial EGF domain is missing or is substituted by one or more different amino acid residues.

Table 1:  A portion (A) of the base sequence of human prourokinase cDNA and
the base sequence (B) for a human prourokinase mutant designed so
as to acquire unique restriction enzyme sites

A

```
-20                                                                          -1
MET ARG ALA LEU LEU ALA ARG LEU LEU LEU CYS VAL LEU VAL VAL SER ASP SER LYS GLY
ATG AGA GCC CTG CTG GCG CGC CTG CTT GTC TGC GTC CTG GTC GTG AGC GAC TCC AAA GGC

 1
SER ASN GLU ELU HIS GLN VAL PRO SER ASN CYS ASP CYS LEU ASN GLY GLY THR CYS VAL
AGC AAT GA(A) CT(T) CAT CAA GTT CCA TCG AAC TGT GAC TGT CTA AAT GGA GGA ACA TGT GTG
            ↓    ↓
            G    C

21
SER ASN LYS TYR PHE SER ASN ILE HIS TRP CYS ASN CYS PRO LYS LYS PHE GLY CLY GLN
TCC AAC AAG TAC TTC TCC AAC ATT CAC TGG TGC AAC TGC CCA AAG AAA TTC GGA GGG CAG

41                                  50
HIS CYS GLU ILE ASP LYS SER LYS THR CYS TYR GLU GLY ASN GLY HIS PHE TYR ARG GLY
CAC TGT GAA ATA GAT AAG TCA AAA ACC T(GC) TAT GAG GGG AAT GGT CAC TTT TAC CGA GGA
                                       ↓
                                       CA

61                                                                          80
LYS ALA SER THR ASP THR MET GLY ARG PRO CYS LEU PRO TRP ASN SER ALA THR VAL LEU --------
AAG GCC AGC ACT GAC ACC ATG GGC CGG CCC TGC CTG CCC TGG AAC TCT GCC ACT GTC CTT --------
```

B

```
-20
MET ARG ALA LEU LEU ALA ARG LEU LEU LEU CYS VAL LEU VAL VAL SER ASP SER LYS GLY
ATG AGA GCC CTG CTG GCG CGC CTG CTT CTC TGC GTC CTG GTC GTG AGC GAC TCC AAA GGC

 1
SER ASN GLU LEU HIS GLN VAL PRO SER ASN CYS ASP CYS LEU ASN GLY GLY THR CYS VAL
AGC AAT GAG CTC CAT CAA GTT CCA TCG AAC TGT GAC TGT CTA AAT GGA GGA ACA TGT GTG
        SacI

21
SER ASN LYS TYR PHE SER ASN ILE HIS TRP CYS ASN CYS PRO LYS LYS PHE GLY GLY GLN
TCC AAC AAG TAC TTC TCC AAC ATT CAC· TGG TGC AAC TGC CCA AAG AAA TTC GGA GGG CAG

41                                  50
HIS CYS GLU ILE ASP LYS SER LYS THR(SER) TYR GLU GLY ASN GLY HIS PHE TYR ARG GLY
CAC TGT GAA ATA GAT AAG TCA AAA ACC TCA TAT GAG GGG AAT GGT CAC TTT TAC CGA GGA
                                       NdeI

61                                                                          80
LYS ALA SER THR ASP THR MET GLY ARG PRO CYS LEU PRO TRP ASN SER ALA THR VAL ELU --------
AAG GCC AGC ACT GAC ACC ATG GGC CGG CCC TGC CTG CCC TGG AAC TCT GCC ACT GTC CTT --------
```

0 253 241

As suitable examples of the missing region in the EGF domain-encoding region, the regions from Asn (asparagine) (l0) to Cys (cysteine) (42); from Asn (l0) to Asp (aspartic acid) (45); and from Asn (l0) to Thr (threonine) (49) were selected in preferred embodiments of the invention. The selection was based on the belief that deletion of such regions might result in changes in the three-dimensional structure since the region from Cys (42) to Thr (49) has a varied physical property, namely from hydrophobic to hydrophilic and then again to hydrophobic.

For the deletion, the so-called protein engineering techniques can be widely used, for example, the site-directed deletion technique (Nucl. Acids Res., ll, l645 (1983)), the site-specific mutagenesis techniques (Craik et al., Science, 228, 29l (l985)), and the technique which employs restriction enzyme treatment and synthetic genes (Wells et al., Gene, 34, 3l5 (l985)).

Table I shows a portion (A) of the base sequence of human prourokinase cDNA prior to the site-specific mutagenesis technique and the base sequence (B) for a human prourokinase mutant obtained by the technique.

The mutant PUK gene resulting from such deletion treatment is inserted into an expression vector system to give a host-vector system for expression. The host-vector system generally comprises the combination of a host and a plasmid vector having a replicon derived from a species compatible with the host cell and a regulatory sequence. The vector generally has a replication site and also has a marker sequence which allows phenotype selection in transformants. For instance, Escherichia coli is transformed generally by using the plasmid pBR322, which is of Escherichia coli strain origin (Bolivar et al., Gene, 2, 95 (l977)). Since it has the ampicillin resistance gene and tetracycline resistance gene, pBR322 give s simple method of detecting transformants. The pBR322 plasmid and other plasmids such as pBR325, pUCl9, pATl53, etc. of microorganism origin have a promoter utilizable by microorganisms for the expression of a protein which is under control of said promoter or has such a promoter sequence inserted therein. Examples of promoters which are generally used in constructing recombinant DNAs include: $\beta$-lactamase (penicillinase) or lactose promoters (Chang et al., Nature, 275, 6l5 (l978); Itakura et al., Science , l98, l056 (l977); Goeddel et al., Nature, 28l, 544 (l979)) and tryptophan (trp) promoters (Goeddel et al., Nucl. Acids Res., 8, 4057 (l979); European patent application laid open under No. 0036776). While these are most commonly used, other microorganism promoters have been discovered and put into use. The detailed base sequences of these have been published (Siebenlist el al., Cell, 20, 269 (l980); Rosenberg et al., Ann. Rev. Genetics, l3, l9 (l979)). It is well known to functionally introduce them into plasmid vectors (Siebenlist el al., Cell, 20, 269 (l980)). As the host, the well-known Escherichia coli strains HBl0l-GCC, C600 and W3ll0 are used, among others.

Suitable examples of the promoters to be used in yeast vectors are the 3-phosphoglycerate kinase (PGK) promoter (Hitzeman et at., Biol. Chem., 255, 2073 (l968) and promoters for other enzymes participating the glycolytic pathway (Hess et al., J. Adv., Enzyme Reg., 7, l49 (l968); Holland et al., Biochemistry, l7, 4900 (l978)), such as enolase, glyceraldehyde-3-phosphate dehydrogenase (GAP-DH), hexokinase, pyruvate decarboxylase, phosphofructokinase, glucose-6-phosphate isomerase, 3-phosphoglycerophosphate mutase, pyruvate kinase, triosephosphate isomerase, phosphoglucose isomerase and glucokinase. Among these, particularly useful are miniaturized GAP-DH promoters and PGK promoters. In constructing an appropriate expression vector, the transcription terminator occurring in these genes is also inserted on the 3' side of the gene desired to be expressed so that the addition of poly(A) to the mRNA and transcription termination can take place. Promoters capable of advantageously controlling transcription according to growth conditions, such as the promoters for the genes for alcohol dehydrogenase 2, isocytochrome C, acid phosphatase, catabolic enzymes involved in nitrogen metabolism, the above-mentioned glyceraldehyde-3-phosphate dehydrogenase, and enzymes in association with the use of maltose or galactose, may also be used. Plasmid vectors containing a yeast-compatible promoter, a replication origin and a transcription terminator can all be used. As the host, the well-known Saccharomyces cerevisiae strains GRFl8 and AH22 are used, among others.

As the secretory expression vector for use in Bacillus subtilis, plasmids having the replication origin of the well-known plasmid pUBll0 and having the promoter, signal peptide gene and terminator for the $\alpha$-amylase gene can be used. Those plasmids containing similar promoter and signal peptide gene as described in Palva et al., Gene, 22, 229 (l983) may also be used. As the host, well-known Bacillus natto as well as well-known Bacillus subtilis can be used, among others.

In recent years, it has become routine to multiply vertebrate animal cells by culture (tissue culture) (Tissue Culture , Academis Press, Kruse and Patterson, editors (1973)). Useful examples of the host cell lines are Vero, HeLa, CHO (Chinese hamster ovary), WI38, BHK, COS-7, MDCK, Cl27, HKG, and human kidney cell lines. The expression vector for use in these cells generally has a replication origin, a promoter located upstream from the gene to be expressed, a ribosome binding site, an RNA splicing site, a poly(A) addition site, and a transcription terminating sequence.

When mammalian cells are used, the regulatory functions on the expression vector are generally of viral origin. For example, the promoters in general use are promoters derived from polyoma. adenovirus 2 or. most frequently, simian virus 40 (SV40). The early and late promoters of SV40 are particularly useful since they can be readily obtained in the form of fragments containing the replication origin of SV40 from the virus (Fiers et al., Nature, 273, II3 (1978)). A fragment of the virus, which comprises about 250 bp from the HindIII site to the BgII site in the replication origin, can be used as well. Furthermore, promoters and regulatory sequences (enhancers) associated with the gene to be expressed can be used provided that they are compatible with the host.

As the promoter-enhancer to be used in expression vectors for use in animal cells, there may be mentioned the SV40 early or late gene promoter-enhancer, the adenovirus major late promoter region, the globulin enhancer-promoter region, the LTR (long terminal repeat) of RNA viruses, the metallothionein promoter region, and the β-actin promoter, among others. As the replication origin, that of the SV40 origin or of another viral (polyoma, adeno, VSV, BPV, etc.) origin may be incorporated in the vector, or the replication mechanism of the host cell chromosome may be used. The latter is sufficient when the vector is incorporated in the host cell chromosome. In addition, a gene amplification system in which the DHFR gene is utilized can be used as another high production system.

While the present invention has been described hereinabove by giving particular examples. it is to be noted that the invention should by no means be construed as being limited to the host cells. vectors and expression systems mentioned above as examples.

In a preferred embodiment of the invention, expression vectors for use in animal cells were constructed by insertion of a gene coding for a human PUK mutant downstream from the SV40 early promoter region. These vectors were incorporated into COS7 cells or CHOKI cells to transform them. In this experimental system, clones capable of producing human PUK mutants at a rate of 50-300 IU/ml/day were obtained.

Human PUK mutants can be purified in the same manner as in purifying known human PUK (Japanese Patent Application (OPI) No. 60-6298I). In a preferred embodiment of the invention, column chromatography using Chelating Sepharose 6B, anti-UK IgG-Sepharose 4B and p-aminobenzamidine-Sepharose 6B combinedly was employed for the purification. Chelating Sepharose 6B is particularly effective in rough purification, anti-UK IgG-Sepharose 4B in high-level purification, and p-aminobenzamidine-Sepharose 6B in removing contaminant active-form urokinase.

Analysis of the thus-obtained product revealed no difference in PUK activity between the mutant form and the non-mutant form. The mutant-form PUK occurred as a single-chain proenzyme having a molecular weight of 45,000 and was completely converted to the active form upon treatment with plasmin. Comparison of the half-life in blood of this human PUK mutant with that of human kidney cell-derived PUK ( J. Biol. Chem., 260, 12377 (1985)) showed that the half-life in blood of the mutant human prourokinase was significantly longer.

The following examples are provided for illustrative purposes only and are in no way intended to limit the scope of the present invention.

## EXAMPLE I

### (A) Construction of expression vector for use in animal cells

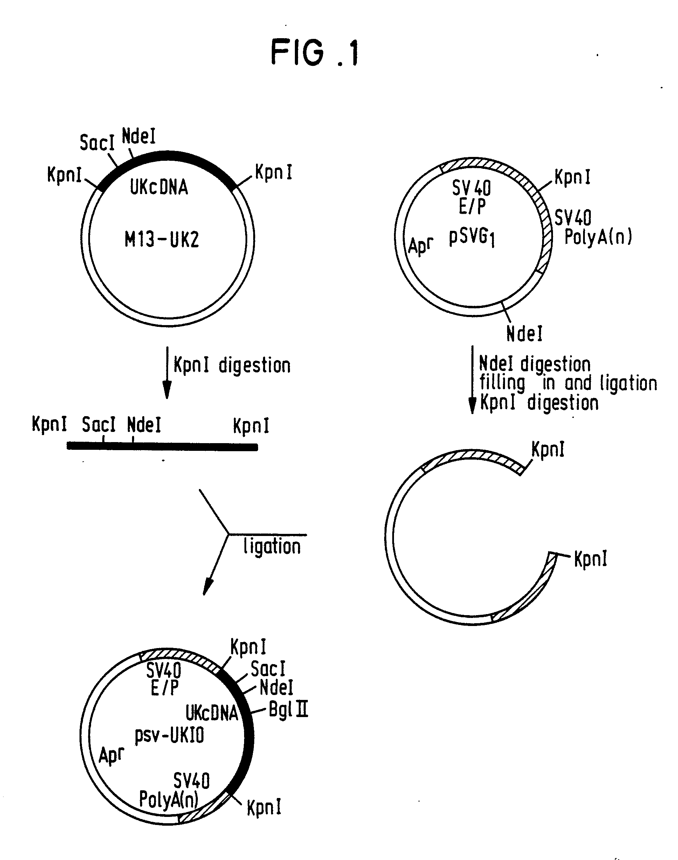

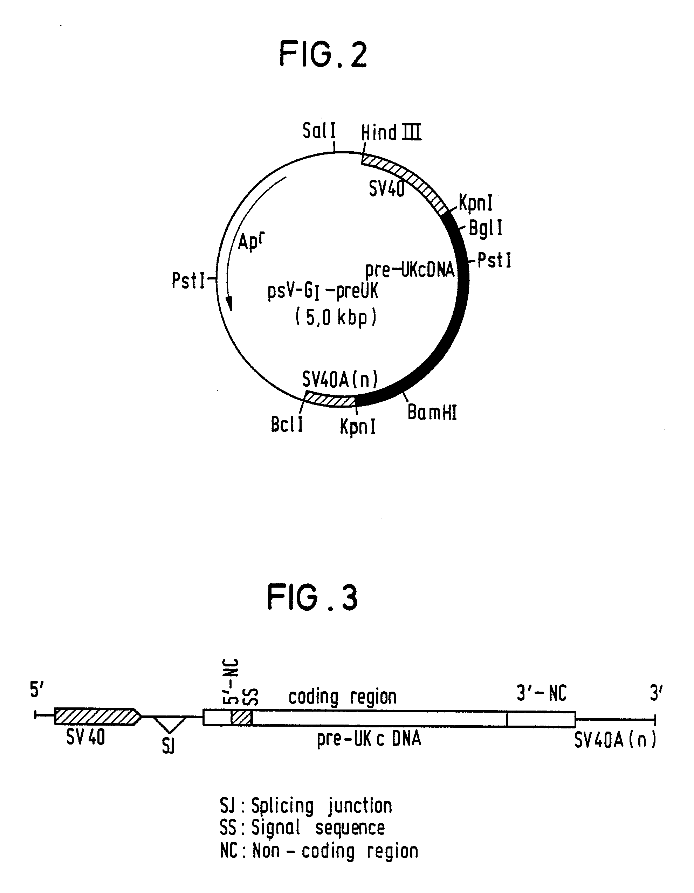

An expression vector, pSV-G₁-preUK, allowing efficient secretory production of human PUK, was constructed (Fig. 2). This vector contains a human PUK cDNA inserted downstream from the SV40 early gene enhancer-promoter region, with the SV40 transcription termination region occurring further downstream from the human PUK cDNA (Fig. 3).

The cloning and sequencing of the PUK cDNA and the construction of the pSV-G₁-preUK plasmid were carried out as described in Japanese Patent Application (OPI) No. 60-18059I or EP-I54272A.

(B) <u>Insertion</u> <u>of</u> <u>preUK</u> <u>cDNA</u> <u>into</u> <u>MI3mpI8RF</u> <u>plasmid</u>

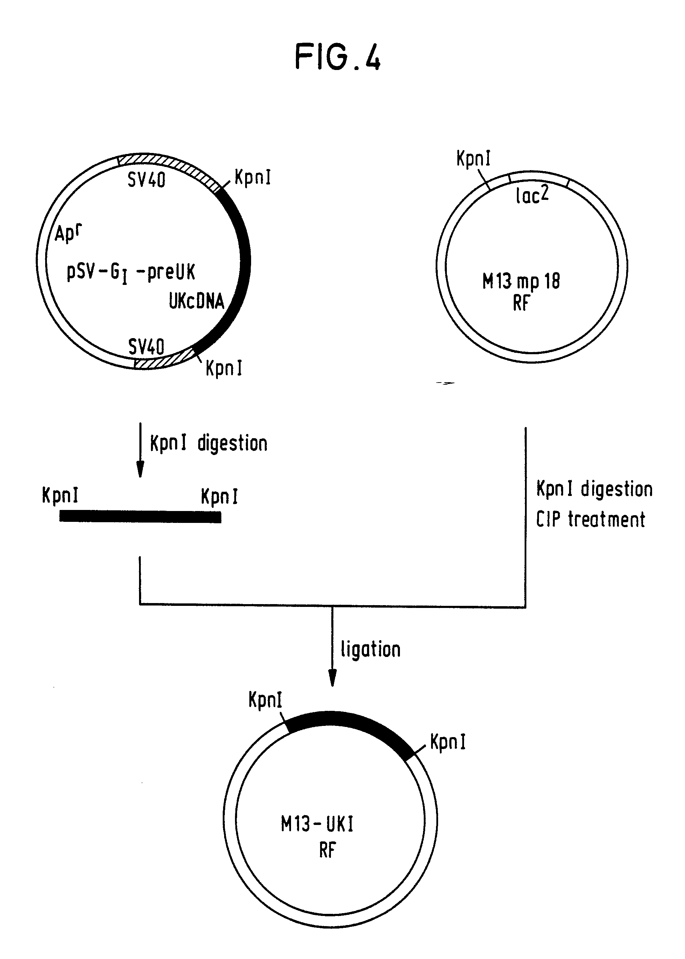

For locating novel unique restriction enzyme sites on the human PUK gene by the technique of site-specific mutagenesis, a KpnI fragment of the plasmid pSV-G₁-preUK was first introduced into the MI3mpI8RF plasmid (Takara Shuzo Co., Ltd.; Messing et al., <u>Method</u> <u>in</u> <u>Enzymology</u>, I0I, 20-78) (Fig. 4).

More specifically, I0 μg of the psV-G₁-preUK plasmid was completely digested with 30 units of the restriction enzyme <u>KpnI</u> at 37°C. This DNA digest was subjected to I% (w/v) agarose gel electrophoresis and a I.7 kb <u>KpnI</u> fragment was recovered from the gel. The techniques used are all as described by T. Maniatis et al. in "Molecular Cloning", Cold Spring Harbor Laboratory (I982). Then, I μg of the MI3mpI8RF plasmid was completely digested with 3 units of <u>KpnI</u> and, then, 5'-end dephosphorylation was effected using calf intestinal alkaline phosphatase (CIP). This <u>KpnI</u>-digested MI3 plasmid was mixed with the I.7 kb <u>KpnI</u> fragment prepared as above and, after addition of 30 units of T4 DNA ligase, the ligation reaction was carried out as described in the above-mentioned technique of Maniatis et al. After the reaction, the resulting DNA was used to transform the <u>Escherichia</u> <u>coli</u> strain JMI05 (Amersham Inc.).

(C) <u>DNA</u> <u>base</u> <u>substitution</u> <u>using</u> <u>the</u> <u>technique</u> <u>of</u> <u>site-specific</u> <u>mutagenesis</u>

Site-specific mutagenesis was effected by the method of Zoller, M.J. et al., <u>DNA</u>, <u>3</u>, 479 (I984) and of wells, J.A. et al., <u>Gene</u>, <u>34</u>, 3I5 (I985).

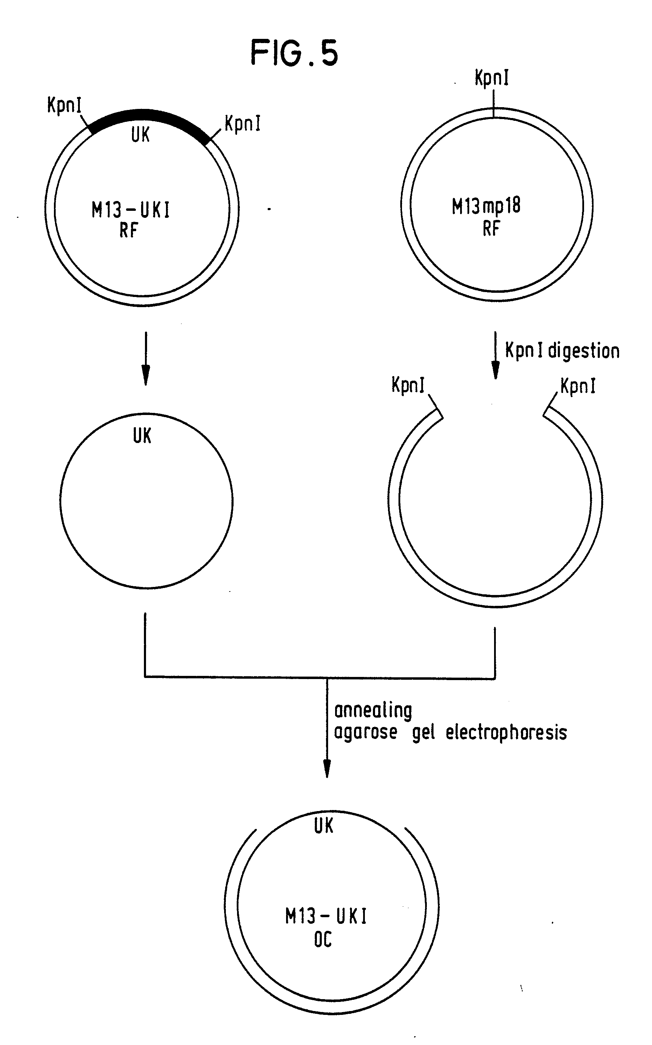

More specifically, a single-stranded DNA was prepared from the <u>Escherichia</u> <u>coli</u> JMI05 transformant containing the MI3-UKIRF plasmid (Fig. 4). This operation was conducted as described. by Sanger, F. et al., <u>J. Mol. Biol.</u>, I43, I6I (I980). Then, I0 μg of this single-stranded MI3-UKI DNA (SS-MI3-UKI) was mixed with I0 μg of the MI3mI8 fragment obtained by <u>KpnI</u> digestion, the mixture was heated to I00°C for 2 minutes, then gradually cooled to 65°C for annealing of SS-MI3-UKI with the negative strand of MI3mI8-<u>Kpn</u> I, and quenched in ice. Thereafter, this DNA was applied to 0.8% (w/v) agarose gel, and the desired double-stranded open-circular-form DNA portion, DNA MI3-UKI-OC (Fig. 5) was recovered.

Then, for base substitution, two oligonucleotides were synthesized. One was a mutagenesis primer for introduction of an <u>SacI</u> site (primer (i)) and the other was a mutagenesis primer for introduction of an NdeI site (primer (ii)). Each was 22mer 2-base-mismatch nucleotide and are shown in Table 2.

### Table 2: Two synthetic oligonucleotide primers

i) Mutagenesis primer for insertion at the <u>SacI</u> site

| | | -1 | 1 | 2 | 3 | 4 | 5 | 6 | 7 | |
|---|---|---|---|---|---|---|---|---|---|---|
| Amino acid | | Gly | Ser | Asn | Glu | Leu | His | Gln | Val | |
| Wild type | 5' − | GGC | AGC | AAT | GAA | CTT | CAT | CAA | GTT | − 3' |
| Mutagenesis primer i) | 3' − | CCG | TCG | TTA | CT C | GA G | GTA | GTT | C | − 5' |

SacI site

ii) Mutagenesis primer for insertion at the <u>NdeI</u> site

| | | 46 | 47 | 48 | 49 | 50 | 51 | 52 | 53 | |
|---|---|---|---|---|---|---|---|---|---|---|
| Amino acid | | Lys | Ser | Lys | Thr | Cys | Tyr | Glu | Gly | |
| Wild type | 5' − | AAG | TCA | AAA | ACC | TGC | TAT | GAG | GGG | − 3' |
| Mutagenesis primer ii) | 3' − | TC | AGT | TTT | TGG | A GT | ATA | CTC | CC | − 5' |

NdeI site

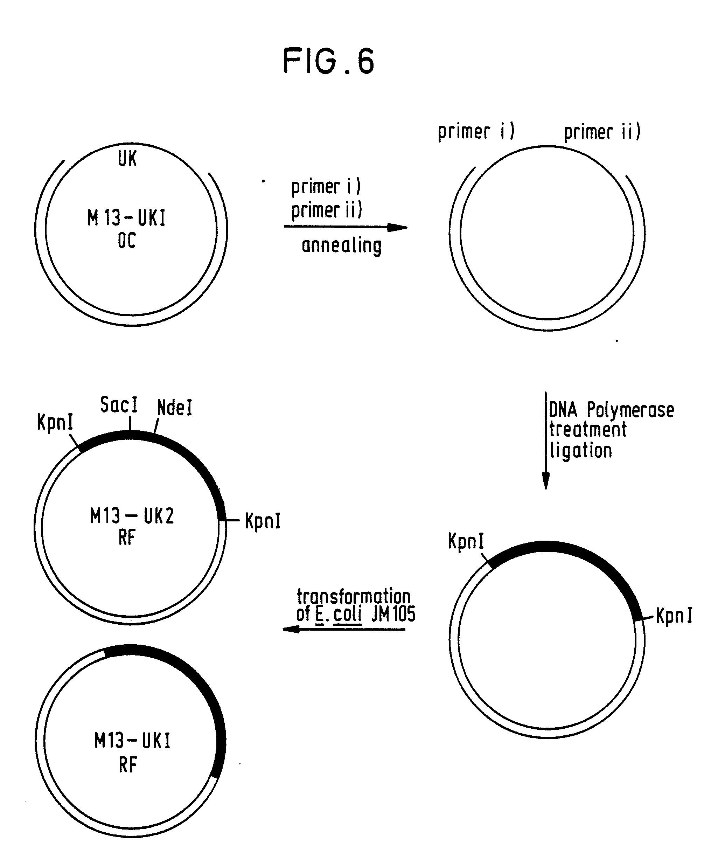

The above two oligonucleotide primers were phosphorylated at the 5′ end using T4 polynucleotide kinase. These two primers (I00 pmol each) were then added, together with I pmol of the MI3-UKI-OC DNA, to annealing buffer (20 mM Tris-HCl, I0 mM MgCl₂, 50 mM NaCl, I mM·DTT, pH 7.5). The mixture was heated at 55°C for I0 minutes and then allowed to stand at room temperature for 20 minutes for annealing of the plasmid with the two primers. Then, an equal volume of Pol I ligation buffer (20 mM Tris-HCl, I0 mM MgCl₂, I0 mM DTT, pH 7.5, 0.5 mM dNTPs, I mM γATP, I0 units T4 DNA ligase, 5 units E. coli DNA polymerase large fragment) was added, and the reaction was carried out at I5°C for 8 hours. This reaction mixture was then used to transform the E. coli JMI05 strain (Fig. 6). For selecting strains carrying the plasmid MI3-UK2RF with the SacI and NdeI sites introduced in the human PUK gene from among a number of transformants, plaque hybridization was carried out using the mutagenesis primers as hybridization probes.

More specifically, 200 to 300 plaques per plate were transferred to a nitrocellulose filter. The nitrocellulose filter was used after immersion in a I00-fold dilution of an overnight E. coli JMI05 culture fluid for 5 minutes, followed by air drying. The resulting filter was placed on H agar with the plaque surface face up and was allowed to stand overnight at 37°C. The phage DNA was then immobilized by the method of T. Maniatis et al., Molecular Cloning, I982. This filter was subjected to prehybridization and hybridization by the method of Zoller, M.J. et al., DNA, 3, 479 (I984). The two mutagenesis primers labeled with (γ-³²p)ATP at the 5′ end were used as the hybridization probes. After hybridization using each probe, washing was performed with 6 × SSC. The washing temperature was raised stepwise to 25°C, 48°C, 56°C and 64°C, whereby the wild type DNA was washed off at 56°C or below while the mutant DNA remained hybridized. As a result of hybridization screening of about I,000 plaques, seven clones remained which hybridized with both the probes even after washing at 64°C. These seven clones were plaque-purified by the method of Zoller, M.J. et al., DNA, 3, 479 (I984). Each plaque was diluted with TE and added in an H agar medium together with an E. coli JMI05 culture fluid. For each clone, I0 plaques were subjected to dot hybridization. In this hybridization, too, the two mutagenesis primers were used as the probes. Then, the DNA base sequence of each clone found positive in dot hybridization was determined by the dideoxy method of Sanger et al., Proc. Natl. Acad. Sci. USA, 74, 5463 (I977). As a result, five clones with the Sac I and NdeI sites introduced therein were obtained. The plasmid of these clones was named MI3-UK2RF (Fig. 6).

### (D) Construction of an expression vector for expression of EGF-domain-deficient human PUK mutant

The plasmid MI3-UK2RF (5 μg) was digested with 20 units of KpnI. Separately, the Nde I site of the plasmid pSVG₁ was eliminated by filling in and, then, I μg of this plasmid was digested with 3 units of KpnI. Both the DNAs were mixed together and the ligation reaction was carried out using I0 units of T4 DNA ligase. This DNA solution was used to transform the Escherichia coli strain HBI0I (Takara Shuzo). From among the resulting transformants (Apʳ), there was obtained a clone, HBI0I/pSV-UKI0, carrying the desired, SacI site-and Nde I site-containing plasmid pSV-UKI0 (Fig. I).

Then, an expression vector for a human PUK mutant deficient in 36 amino acid residues for Asn-I0 to Asp-45 was constructed using the plasmid pSV-UKI0. Since digestion of pSV-UKI0 with the restriction enzymes SacI and NdeI results in elimination from the human PUK gene of the codons for the 4th amino acid (leucine) from the N terminus thereof to the 50th amino acid (cysteine) (Table 3A), oligonucleotides (Table 3B) were synthesized to give a DNA fragment coding for His-5 to Ser-9 plus Lys-46 to Cys-50 and having an SacI and an NdeI digestion terminus.

Table 3: SacI-NdeI digestion/cleavage region (A) of pSV-UK10 and synthetic oligonulceotide (B) for insertion at said site

```
        -20
A       MET ARG ALA LEU LEU ALA ARG LEU LEU LEU CYS VAL LEU VAL VAL SER ASP SER LYS GLY
------- ATG AGA GCC CTG CTG GCG GGC CTG CTT CTC TGC GTC CTG GTC GTG AGC GAC TCC AAA GGC

        1   2   3   4
        SER ASN GLU LEU
        AGC AAT GAG CT
        TCG TTA C

                                                   51                                  60
                                                   TYR GLU GLY ASN GLY HIS PHE TYR ARG GLY
                                                   TAT GAG GGG AAT GGT CAC TTT TAC CGA GGA
                                                 A CTC CCC TTA CCA GTG AAA ATG GCT CCT

        61
        LYS ALA SER THR ASP THR MET GLY ARG PRO CYS LEU PRO TRP ASN SER ALA THR VAL LEU
        AAG GCC AGC ACT GAC ACC ATG GGC CGG CCC TGC CTG CCC TGG AAC TCT GCC ACT GTC CTT

        81                                                                          100
        GLN GLN THR TYR HIS ALA HIS ARG SER ASP ALA LEU GLN LEU GLY LEU GLY LYS HIS ASN -------
        CAG CAA AGG TAC CAT GCC CAC AGA TCT GAT GCT CTT CAG CTG GGC CTG GGG AAA CAT AAT -------

B       3   4   5   6   7   8   9   46  47  48  49  50
       (GLU LEU) HIS GLN VAL PRO SER LYS SER LYS THR CYS

5' -          C  CAT CAA GTT CCA TCG AAG TCA AAA ACC TGC     - 3'
3' -   TC GAG    GTA GTT CAA GGT AGC TTC AGT TTT TGG ACG AT - 5'
```

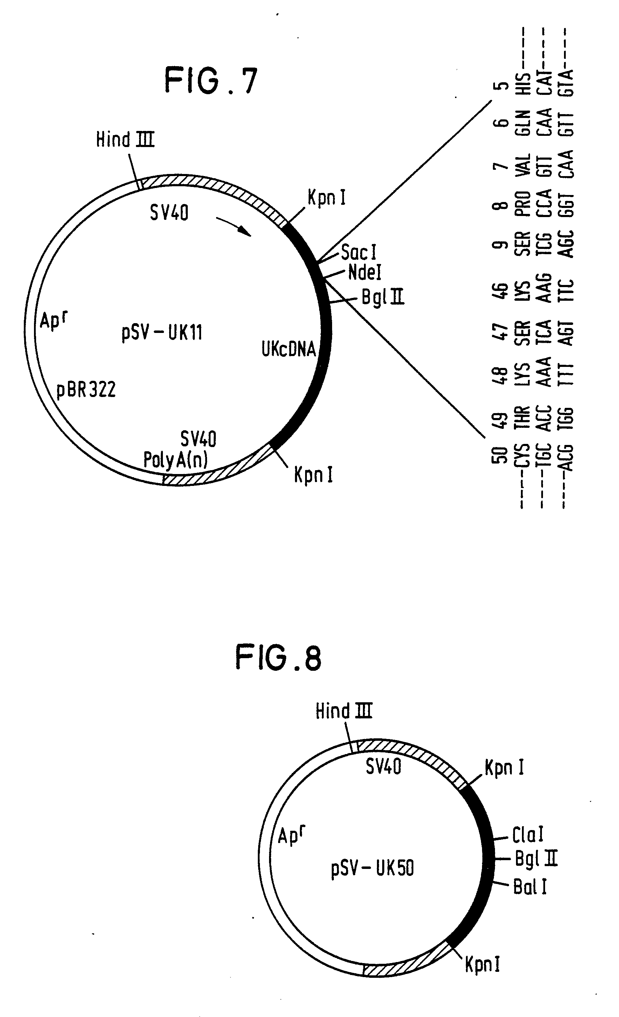

The 3I mer and 37 mer oligonucleotides synthesized (each 50 pmol) were mixed together, and the mixture was heated at 70°C and then allowed to stand at room temperature for 20 minutes for annealing. The resulting DNA and I μg of the SacI-NdeI digest fragment of the plasmid pSV-UKI0 were mixed together and ligated using 30 units of T4 DNA ligase. As a result, a vector, pSV-UKII, allowing expression in animal cells of a human PUK mutant deficient in the l0th amino acid (asparagine) residue from the N terminus of human PUK to the 45th amino acid (aspartic acid) residue was constructed (Fig. 7).

In the same manner, various synthetic genes were inserted in the SacI-NdeI site and expression vectors for human PUK mutants characterized by various forms of variation (deletion, and substitution of amino acid residue) within the region from Leu-4 to Cys-50 of human PUK were constructed.

## (E) Construction of derivatives within the kringle domain of human PUK

Using the same technique of site-specific mutagenesis as mentioned above, ATA (Ile-44) was converted to ATCand, as a result, an expression vector, pSV-UK50, having a ClaI restriction enzyme site within the DNA base region coding for Ile-44 to Asp-45 was constructed (Fig. 8).

Digestion of pSV-UK50 with Cla I and BglII, ClaI and BalI, or BglII and BalI followed by insertion, at the resulting site, of a variety of synthetic oligonucleotides in the same manner as above led to construction of expression vectors for human PUK mutants modified within the kringle domain.

## (F) Production of human PUK mutants in animal cells

The expression vectors for human PUK mutants were introduced onto the chromosome of Chinese hamster ovary cells (CHO-K$_1$, ATCC CCL6I) using pSV-G$_1$-Neo was used as a dominant selective marker. The construction of pSV-G$_1$-Neo, cotransfection of CHO-K$_1$ cells with the same together with the human PUK mutant expression vector and cloning were performed as described in Japanese Patent Application (OPI) No. 60-I8059I.

A clone, CHO-UKII, as transformed with the plasmid pSV-UKII (deficient in codons for Asn-I0 to Asp-45) was cultured and the culture supernatant was assayed for plasminogen activator activity. Thus, when first subjected to fibrin-agar plate assay, the supernatant was found active. The activity was neutralized by anti-human urokinase antibody. Further screening gave a strain, CHO-UKII-7, seemingly higher in productivity of the human PUK mutant. Using the culture supernatant of this clone, the properties of the mutant human PUK protein produced were investigated.

## (G) Purification of the human PUK mutant produced in the cell line CHO-UKII-7

CHO-UKII-7 cells suspended in 5 ml of Ham's F-I2 medium containing 5% (w/v) of fetal calf serum were placed in a 25-cm$^3$ culture flask (Corning #25I00) (5 $\times$ I0$^{-5}$ cells per flask). After 3 days of incubation, when the culture was confluent, the cells were washed with several portions of Ham's F-I2 medium (without addition of fetal calf serum). Thereafter, Ham's F-I2 medium containing aprotinin (I0 KIU/ml) and 0.1% (w/v) human serum albumin was used as a maintenance medium, with the culture medium exchanged at 2-to 3-day intervals. The mutant human PUK was purified from I liter of this serum-free culture supernatant. More specifically, the culture supernatant was adjusted to pH 5.5 by adding IN HCl and, then, a suspension of CM-Sephadex C-50 (Pharmacia) (in an amount corresponding to I.5 g on the dry basis) in 0.I6 M sodium phosphate (pH 5.5) was added. The mixture was stirred at 4°C for 2 hours. The gel was recovered on a glass filter, washed with 0.I6 M sodium phosphate (pH 5.5), and packed into a column (2.5 $\times$ I0 cm). Elution was carried out with 0.I6 M sodium phosphate (pH 8.5). An active eluate fraction showing fibrinolytic activity on a fibrin-agar plate (J. Cell Biol., 94, 63I (I982)) was poured into a column (I.5 $\times$ I0 cm) packed with Sepharose 4B (gel volume: 9 ml) with anti-urokinase polyclonal antibody (freed from protease activity by passage through DEAE Affi-Gel Blue, p-Aminobenzamidine-Sepharose or Lysine-Sepharose) immobilized thereon. After washing the antibody column with 0.5 M NaCl-0.I M sodium phosphate (pH 7.0), elution was carried out with 0.2 M glycine-HCl-0.5 M NaCl (pH 2.5). An active fraction showing fibrinolytic activity on a fibrin-agar plate was recovered, adjusted to pH 7.0 by dropwise addition of I M aqueous Tris, and subjected to the subsequent analysis of its properties:

## (I) Molecular weight

The molecular weight of the CHO-UKII-7 cell-derived human PUK mutant was determined by the SDS-polyacrylamide gel electrophoretic method described in Nature, 227, 680 (1970). Under reducing as well as non-reducing conditions, the molecular weight was found to be about 45 kilodaltons. The above result shows that the CHO-UKII-7 cell-derived human PUK mutant has a single-chain structure with a molecular weight of about 45 kilodaltons.

## (2) Enzyme susceptibility

The urokinase activity of the CHO-UKII-7 cell-derived human PUK mutant was determined by measuring the amidase activity against the synthetic substrate S-2444 (Kabi) for a urokinase activity assay. Human urine-derived urokinase was used as a control. More specifically, a I μg/ml solution of the CHO-UKII-7 cell-derived human PUK mutant or human urine-derived urokinase in 0.05 M Tris-HCl, 0.038 M NaCl 0.2% (w/v) BSA (pH 8.8) was prepared and treated with plasmin in known concentrations at 37°C for I hour, and the urokinase activity thus expressed was determined using the synthetic substrate S-2444 (J. Biol. Chem., 260, 12377 (1985)). The CHO-UKII-7 cell-derived human PUK mutant itself did not show urokinase activity. However, plasmin treatment resulted in expression of urokinase activity (Table 4).

**Table 4: Urokinase activity after plasmin treatment (IU/ml)**

|  | No plasmin treatment | Plasmin concentration (mCU/ml) | | | |
|---|---|---|---|---|---|
|  |  | 0.375 | 1.5 | 6 | 12 |
| Human urine urokinase | 151 | 153 | 149 | 152 | 150 |
| CHO-UK11-7 cell-derived mutant human PUK | 0.6 | 91 | 122 | 140 | 138 |

This enzyme activity was destroyed by anti-urokinase polyclonal antibody. This revealed that the CHO-UKII-7 cell-derived human PUK mutant is an enzyme which is in an inactive form but is capable of expressing urokinase activity upon plasmin treatment.

## EXAMPLE 2

### (A) Construction of EGF domain-deficient mutant human PUK genes

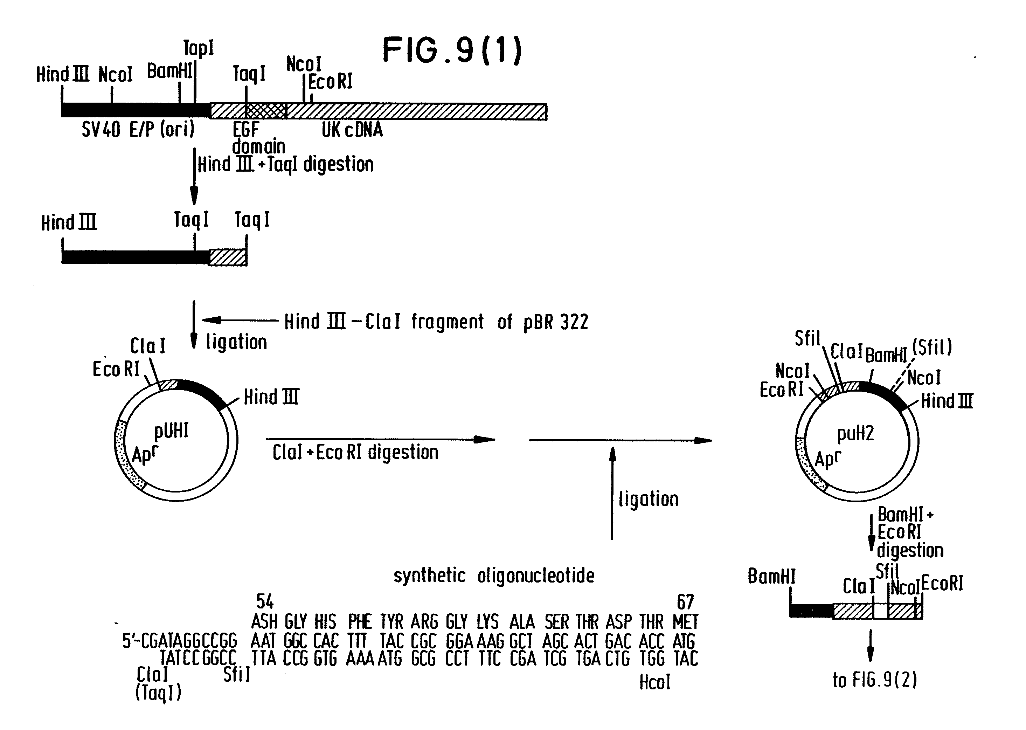

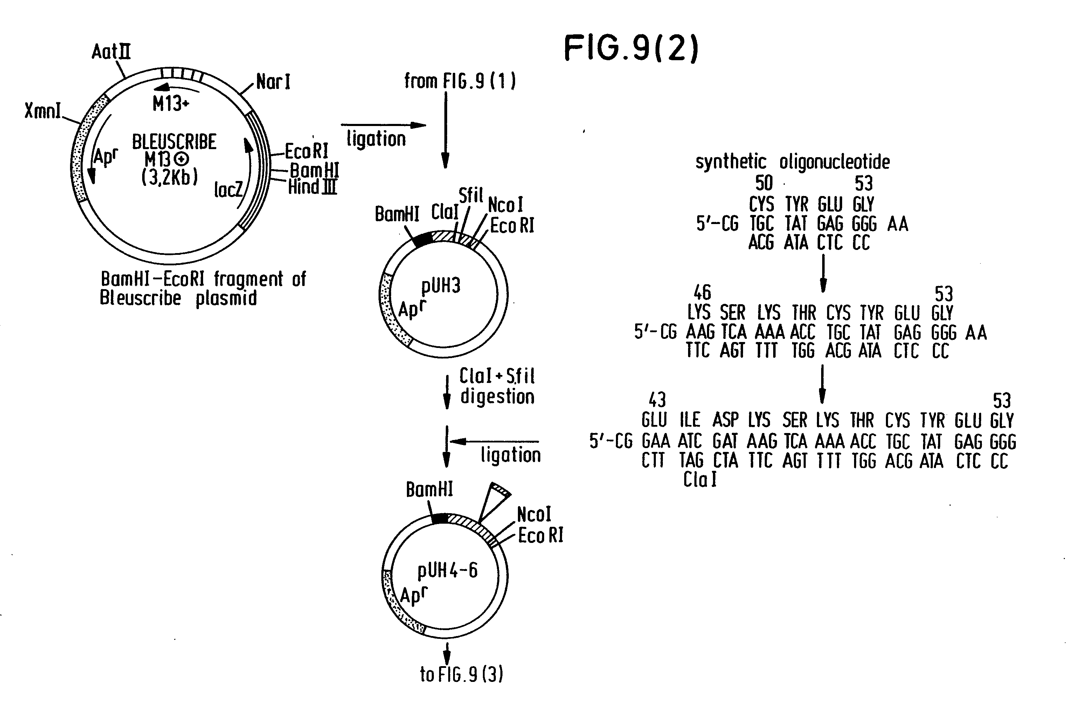

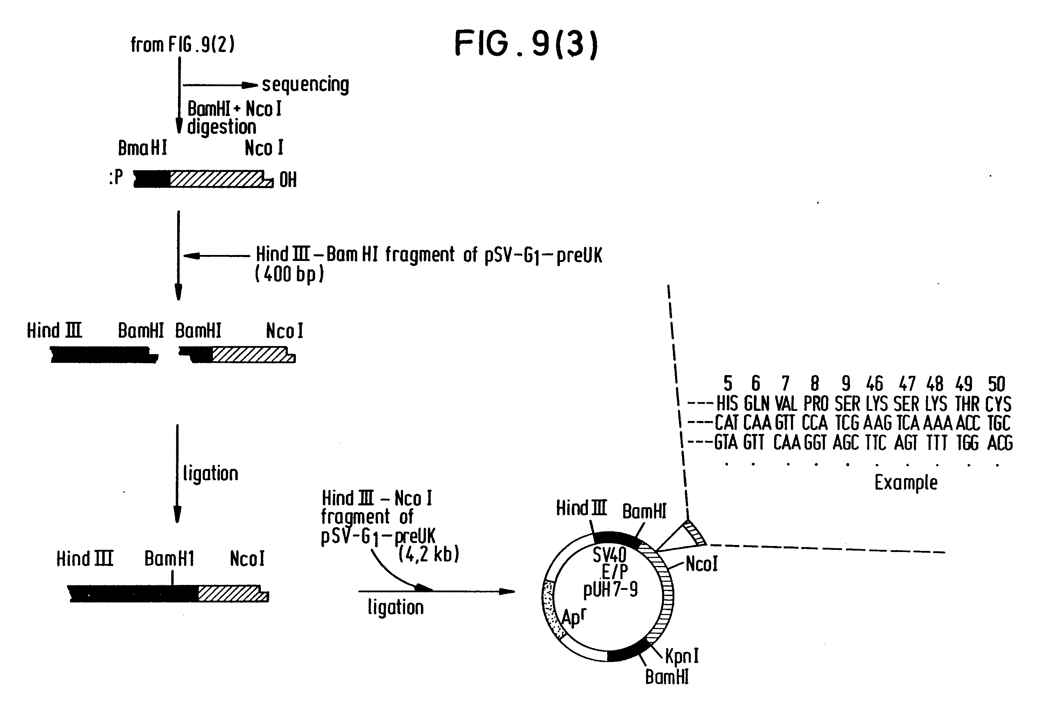

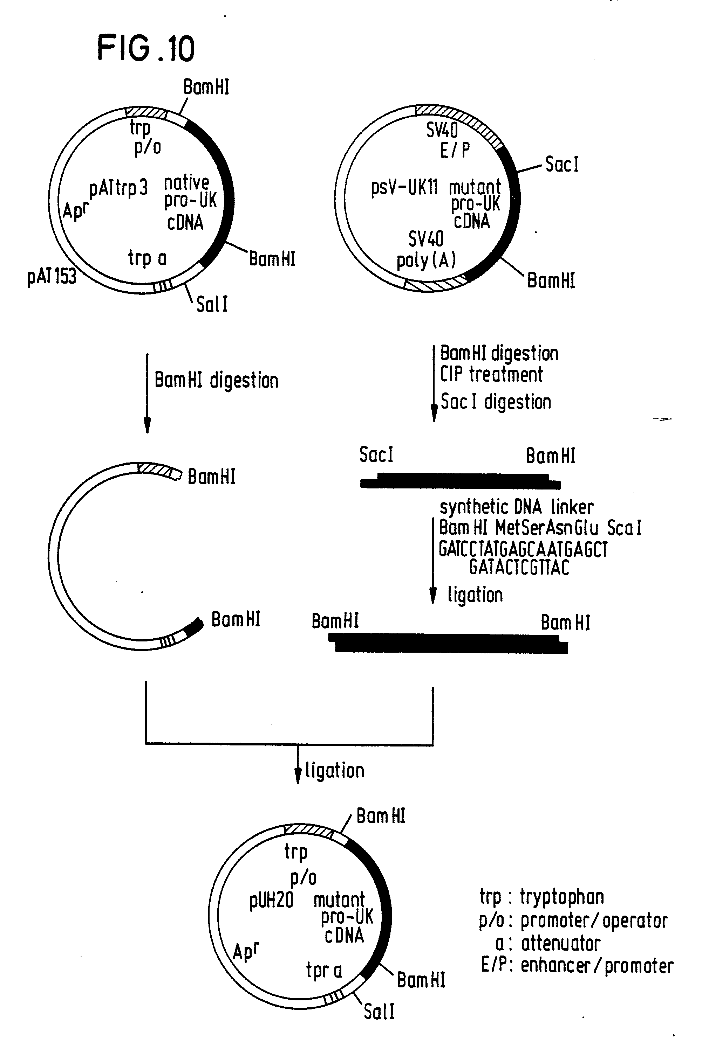

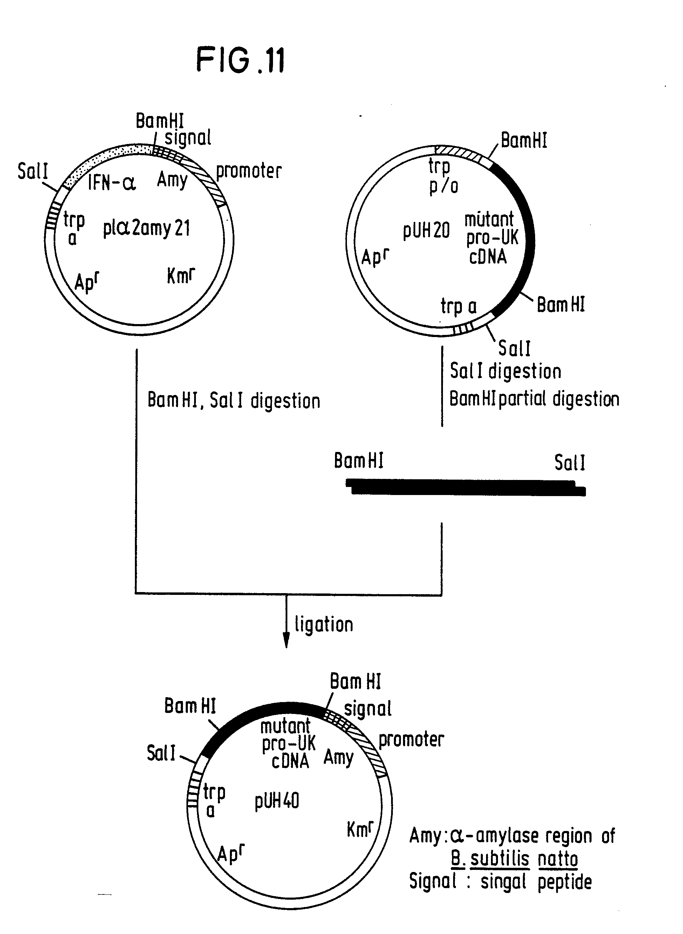

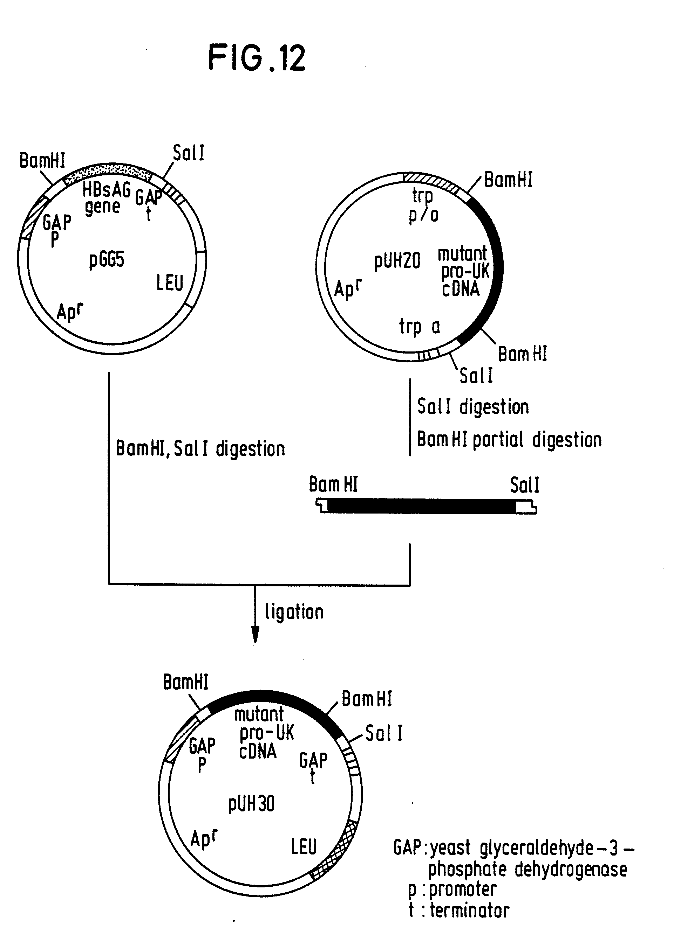

Three expression vectors for human PUK deficient in EGF domain regions of Asn-I0 to Cys-42, Asn-I0 to Asp-45, and Asn-I0 to Thr-49, respectively, were constructed. The method of deleting each EGF domain-encoding region from the human PUK gene and the construction schemes for the respective expression vectors for use in animal cells are outlined in Fig. 9.

Thereafter, the EGF domain encoding regions from the human PUK gene were eliminated by utilizing the NcoI and TaqI sites proximal to said regions. More specifically, the expression vector for human PUK pSV-GI-preUK was digested with HindIII and TaqI, and a partial human PUK gene fragment containing the coding region for the N terminus to the I0th amino acid residue (Asn) was isolated and inserted into the plasmid pBR322, whereby the plasmid pUHI was constructed. Then, a synthetic gene coding for the region from Asn-54 to Met-67 of human PUK and having ClaI and EcoRI sites at the termini thereof was prepared and inserted into the plasmid pUHI to give the plasmid pUH2. this synthetic gene was also provided with an SfiI site thereon.

The BamHI-EcoRI fragment containing only the Sfil site on the synthetic gene was inserted into the BLUESCRIBE plasmid (Vector Cloning). Thus was constructed, the plasmid pUH3. Three plasmids (pUH4 to pUH6) having the partial human PUK (N terminus to Met-67) genes deprived of the respective EGF domain regions were constructed by inserting three synthetic genes coding for Cys-50 to Gly-53, Lys-46 to Gly-53, and Glu-43 to Gly-53, respectively and having a ClaI and an Sfil site at the respective ends into pUH3 at the Sfil-ClaI site. Then, for inserting these EGF domain-deficient regions into an expression vector, the BamHI-NcoI fragment of each of pUH4 to pUH6 and the Hin dIII-BamHI fragment of pSV-preUK were joined together. The resulting fragment was inserted into pSV-GI-preUK in the HindIII-NcoI region. In this manner, three expression vectors, pUH7 to pUH9, for the corresponding EGF domain-deficient human PUK mutants were constructed. The construction of these expression vectors is described below in further detail.

In this example, unless otherwise specified, each DNA digestion with a restriction enzyme or enzymes was carried out overnight at 37°C. The digestion of DNA prepared by the alkaline denaturation (mini-prep) method was performed using 5 units of restriction enzyme per microgram of DNA. The buffer compositions for restriction enzymes were as recommended by the enzyme suppliers. The ligation reaction was carried out at 16°C for 1 to 2 hours using DNA ligation kits (Takara Shuzo) and the following reaction mixture composition:

| DNA fragment | 5.0 µl (500 ng) |
|---|---|
| Vector DNA | 2.5 µl (250 ng) |
| Buffer A [1] | 60.0 µl |
| Buffer B [2] | 7.5 µl |
| | 75.0 µl |

1) Takara ligation kit reaction buffer A

2) Takara ligation kit enzyme solution B

The DNA base sequence determination was made by the dideoxy sequencing method using the plasmid obtained by the alkaline denaturation method.

(I) Synthesis of oligonucleotides

Nine oligonucleotides having oligonucleotides sequences shown in Table 5 below were synthesized using an automatic DNA synthesizer.

Table 5:  Sequence of oligonucleotides synthesized by automated DNA synthesizer

| Length (-mer) | Sequence (5'->3') | Used |
|---|---|---|
| 11 | CCCTCATAGC A | Synthetic gene |
| 16 | CGTGCTATGA GGGGAA | Synthetic gene |
| 18 | GAACTTCATC AAGTTCCA | Sequencing primer |
| 23 | CCCTCATAGC AGGTTTTTGA CTT | Synthetic gene |
| 28 | CGAAGTCAAA AACCTGCTAT GAGGGGAA | Synthetic gene |
| 32 | CCCTCATAGC AGGTTTTTGA CTTATCGATT TC | Synthetic gene |
| 37 | CGGAAATCGA TAAGTCAAAA ACCTGCTATG AGGGGAA | Synthetic gene |
| 54 | CGATAGGCCG GAATGGCCAC TTTTACCGCG GAAAGGCTAG CACTGACACC ATGG | Synthetic gene |
| 56 | AATTCCATGG TGTCAGTGCT AGCCTTTCCG CGGTAAAAGT GGCCATTCCG GCCTAT | Synthetic gene |

One of them was used as a sequencing primer and the others were all used as synthetic genes. The oligonucleotides synthesized were treated for deprotection and then separated and purified by denaturing gel electrophoresis. A portion of each purified oligonucleotide was labeled with $^{32}$P at the 5' end for purity checking. Each oligonucleotide was observed as a single band irrespective of size.

(2) Annealing

Four synthetic genes shown in Table 6 were prepared by annealing each pair of synthetic oligonucleotides complementary in sequence to each other.

0 253 241

Table 6:

|  | Gene | Annealed* DAN length |
|---|---|---|

```
                          54                                              67
                          ASN GLY HIS PHE TYR ARG GLY LYS ALA SER THR ASP THR MET
        5'- CGATAGGCCGG   AAT GGC CAC TTT TAC CGC GGA AAG GCT AGC ACT GAC ACC ATG G      56-mer
             TATCCGGCC    TTA CCG GTG AAA ATG GCG CCT TTC CGA TCG TGA CTG TGG TAC GTTA   54-mer
        ClaI        SifI                                    NheI          NcoI   EcoRI
        (TaqI)


                          43                                   53
                          GLU ILE ASP LYS SER LYS THR CYS TYR GLU GLY
              5' - CG     GAA ATC GAT AAG TCA AAA ACC TGC TAT GAG GCG AA                 37-mer
                          CTT TAG CTA TTC AGT TTT TGG ACG ATA CTC CC                     32-mer
                              ClaI


                              46                               53
                              LYS SER LYS THR CYS TYR GLU GLY
                 5' - CG     AAG TCA AAA ACC TGC TAT GAG GGG AA                          28-mer
                             TTC AGT TTT TGG ACG ATA CTC CC                              23-mer


                                  50              53
                                  CYS TYR GLU GLY
                     5'- CG      TGC TAT GAG GGG AA                                      16-mer
                                 ACG ATA CTC CC                                          11-mer
```

This annealing reaction was effected by heating at 75°C for l0 minutes followed by gradual cooling in 20°C over 3 hours. The reaction mixture composition used was as follows:

| | |
|---|---|
| Oligonucleotide a | 10 µl (100 pmol) |
| Oligonucleotide b | 10 µl (100 pmol) |
| 10 x buffer A * | 3 µl |
| Redistilled water | 7 µl |
| | 30 µl |

$$* \; 10 \; x \; buffer \; A: \quad 100 \; mM \; Tris-HCl, \; pH \; 7.6$$
$$5 \; mM \; MgCl_2$$

An equimolar mixture of the 56mer and 54mer oligonucleotides each labeled with $^{32}$P at the 5′ end was gradually cooled from 75°C to 20°C and the degree of annealing was examined by electrophoresis. Free single-stranded DNAs were almost absent and the DNAs were mostly in the double-stranded form. Therefore, other oligonucleotides were subjected to annealing under the same conditions and the partial genes thus constructed were inserted into various plasmids.

(3) Construction of the plasmids pUHl to pUH3

Since a number of TaqI sites are present on the plasmid pSV-Gl-preUK, it is difficult to cause cleavage only at the TaqI site proximal to the EGF domain by one restriction enzyme treatment alone. Therefore, a DNA fragment containing this TaqI site was excited. More specifically, pSV-Gl-preUK was digested with the restriction enzymes HindIII and BgIII and the resulting DNAs were examined by agarose gel electrophoresis. A l.l kb DNA fragment containing the EGF domain-encoding region was recovered from the corresponding gel portion excised from the gel. Then, this fragment was partially digested with TaqI and the digest was subjected to polyacrylamide gel electrophoresis. After confirmation of the presence of the desired 760 bp band, this DNA fragment was recovered.

Separately, the plasmid pBR322 was digested with HindIII and ClaI, whereby a 4.3 kb DNA fragment was obtained. This DNA was ligated with the 760 bp Hin dIII-TaqI DNA fragment prepared previously. This ligation caused conversion of the TaqI site originally occurring at the 3′ end of the 760 bp fragment to a ClaI site. The reaction mixture was used to transform the E. colistrain HBlOl. The DNAs prepared from 24 transformant strains were each digested with ClaI and NcoI. The desired plamid pUHl (Fig. 9) was expected to be cleaved with either ClaI or NcoI only at one site. Electrophoresis revealed 6 clones to be the desired plasmid.

Then pUHl was digested with Eco RI and ClaI, followed by agarose gel electrophoresis. An about 5.0 kb DNA fragment was recovered from the gel, and this DNA was ligated with the annealing product from the 56mer and 54mer synthetic oligonucleotides prepared in steps (l) and (2) men tioned above. The reaction mixture was used to transform E. coli HBlOl, DNAs prepared fom 20 transformant strains were digested with NcoI and EcoRI-HindIII. The desired plasmid pUH2 would give an about 700 bp DNA fragment upon digestion with NcoI and an about 8l0 bp DNA fragment upon EcoRI-HindIII digestion. Electrophoresis revealed 4 clones to be the desired plasmid.

Since pUH2 contains two SfiI sites in close vicinity to each other, the BamHI-EcoRI fragment containing the synthetic gene-drived SfiI site was transferred to another vector. Thus, pUH2 was first digested with BamHI and Eco RI. The digest was subjected to polyacrylamide gel electrophoresis. A 330 bp DNA fragment was recovered from the corresponding gel portion by electroelution.

Separately, the plasmid BLUESCRIBE (Vectorcloning Systems) was digested with BamHI and EcoRI, and the about 3.l kb DNA fragment obtained was ligated with the 330 bp Bam HI-EcoRI fragment prepared previously, and this reaction mixture was used to transform the E. coli strain JMl05. Twelve strains were selected from among the transformants obtained and the DNA prepared from each of the twelve strains was digested with Sfi I and ClaI. If the DNA is the desired plasmid pUH3, it is cleaved only at one site with each of Sfil and Cla I. As a result of electrophoresis, eight clones were found to carry the desired plasmid.

(4) Construction of the plasmids pUH4 to pUH9

An attempt ws made to construct the plasmids pUH4 to pUH6 having partial genes for human PUK deprived of the EGF domain by inserting the three synthetic genes into the plasmid pUH3 in the ClaI-Sfil, and an about 3.l kb DNA fragment was recovered by agarose gel electrophoresis followed by electroelution. This fragment was ligated with each of the three synthetic genes. These reaction mixtures were used to transform the E. coli strain JMl05. For each synthetic gene, l0 strains were selected from among the transformants obtained, and the DNA prepared form each strain was digested with BamHI and EcoRI. If the DNA is the desired plasmid pUH4, pUH5 or pUH6, it gives, upon restriction enzyme digestion, a 360 bp fragment (in the case of pUH4), a 2350 bp fragment (in the case of pUH5) or a 340 bp fragment (in the case of pUH6). As a result of checking by polyacrylamide gel electrophoresis, 2 clones of pUH4, 2 clones of pUH5 and 3 clones of pUH6 were obtained.

Using these plasmids, the DNA base sequences in the EGF domain region were determined. As a result, it was found that the regions intended for deletion had been deleted without causing any change in the remaining amino acid sequence.

Then, the SV40 early gene promoter region-EGF domain-deficient human PUK mutant-encoding partial gene portion was excised from each of the plasmids pUH4 to pUH6 and incorporated in the human PUK expression vector pSV-Gl-preUK for use in animal cells. More specifically, pUH4 to pUH6 were cleaved with Nco I and then subjected to CIP treatment for dephosphorylation at the 5' end. These DNAs digested with BamHI, the digests were subjected to polyacrylamide gel electrophoresis, and a 360 bp DNA fragment (from pUH4), a 350 bp DNA fragment (from pUH5) and a 340 bp DNA fragment (from pUH6) were recovered by electroelution from the corresponding gel segments excised from the polyacrylamide gel. These DNAs were each ligated with a 400 bp BamHI-HindIII fragment of the plasmid pSV-Gl-preUK as separately prepared. After the ligation reaction, DNAs were recovered by precipitation with ethanol.

Separately, pSV-Gl-preUK was digested with HindIII and NcoI and the digest was subjected to electrophoresis. An about 4.2 kb DNA fragment segment was excised from the gel and the DNA was recovered by electroelution. This 4.2 kb HindIII-NcoI DNA fragment was religated with the above-mentioned three ligation samples. Each reaction mixture was used to transform the E. coli strain HBl0l. The transformants containing plasmids pUH7, pUH8 and pUH9 were deposited at Fermentation Institute of Osaka (IFO) in Osaka, Japan on July 2, l987 under accession numbers IFO l4636, l4637 and l4638, respectively. For each reaction mixture l6 strains were selected from among the transformants obtained and the DNA extracted from each strain was digested with HindIII and with HindIII and BglII. If the DNA is the plasmid pUH7, pUH8 or pUH9, HindIII diges tion must give an about 4.9 kb DNA fragment and HindIII-BlgII digestion must give an about l.0 kb fragment. As a result of electrophoresis, 3 clones of pUH7, 3 clones of pUH8 and 5 clones of pUH9 were obtained. For each plasmid, one clone was selected and used for large quantity preparation of the plasmid. The structures of the plamids thus obtained were analyzed in further detail (Table 7).

Table 7: Restriction enzyme treatment of human PUK mutant expression vectors

| Plasmid | Fragment size (Kb) | | | | | |
|---|---|---|---|---|---|---|
| | ClaI | HindIII | BamHI | KpnI | HindIII + BglII | BglII + KpnI |
| pUH7 | 4.9 | 4.9 | 3.1, 1.3, 0.3 | 3.5, 1.4 | 3.9, 1.0 | 3.5, 0.4, 1.0 |
| pUH8 | 4.9 | 4.9 | 3.1, 1.3, 0.3 | 3.5, 1.4 | 3.9, 1.0 | 3.5, 0.4, 1.0 |
| pUH9 | 4.9, 0.8 | 4.9 | 3.1, 1.3, 0.3 | 3.5, 1.4 | 3.9, 1.0 | 3.5, 0.4, 1.0 |
| pSVG1-UK | 5.0 | 5.0 | 3.1, 1.4, 0.3 | 3.5, 1.5 | 3.9, 1.1 | 3.5, 0.5, 1.0 |

pUH7: Deficient in the region Asn(10)-Cys(42)
pUH8: Deficient in the region Asn(10)-Asp(45)
pUH9: Deficient in the region Asn(10)-Thr(49)

The sizes of fragments obtained by digestion with a variety of restriction enzymes perfectly agreed with the anticipated values. In this way, three expression vectors for EGF domain-deficient human PUK mutants, pUH7 to pUH9, were constructed.

Based on the results of measurement of the above-mentioned restriction enzyme digest fragments, it is believed that no substantial deletion is found in other parts than the EGF domain. Sequencing of the neighborhood of the deleted portion failed to reveal any change in base sequence. In view of the above, it is believed that no deletion or mutation occurred at places other than the region in question.

(5) Production of EGF domain-deficient human PUK mutants in COS7 cells

The three expression vectors pUH7 to pUH9 were each introduced, in an amount of 20 μg, into COS7 cells (3 × 10⁶ cells), and the activity of each expression product and the productivity were investigated (Table 8).

Table 8: Production of human PUK mutants by COS7 cells

| Cells Cultured | Serum-free medium | | 10% Fetal calf serum-containing medium | |
|---|---|---|---|---|
| | Fibrin plate method (IU/ml) | RPHA method (units/ml) | Fibrin plate method (IU/ml) | RPHA method (units/ml) |
| pUH7 | 180 | 200 | 140 | 150 |
| pUH8 | 180 | 150 | 120 | 150 |
| pUH9 | 160 | 150 | 140 | 150 |
| pSV-G1-preUK | 120 | 120 | 80 | 100 |

(Human urinary urokinase, 1,150 IU/ampoule, was used as a reference standard.)

More specifically, after DNA introduction, the COS cells were cultured in D-MEM (Nissui Seiyaku) containing l0% (w/v) FCS (GIBCO) or in a serum-free medium containing l0 KIU/ml of aprotinin (SIGMA). In each case, the culture was samples after 72 hours of incubation and assayed for plasminogen activator activity by the fibrin-agar plate method and for antigen quantity by the RPHA assay method. For each clone and irrespective of the presence or absence of serum, the production was about 200 IU/ml and there was no substantial difference between the biological activity value and the activity value calculated on the basis of the antigen quantity. The above findings demonstrate that deletion of the EGF domain does not influence the expression of plasminogen activator activity.

### (6) Introduction of expression vectors for EGF domain-deficient human PUK mutants into CHO cells

The three human PUK mutant expression vectors were introduced into CHO KI cells (ATCC CCL6I).

More specifically, the plasmids pUH7 to pUH9 were introduced into CHO KI cells together with pSV-GI-Neo by the DNA-calcium phosphate precipitate method. The cells were cultured in a medium containing G4I8-resistant colonies obtained were transferred to 96-well plates by the cloning cylinder method. The efficiency of plasmid introduction was $1.2 \times 10^{-5}$ colonies/μg/dish for pUH7, $3.2 \times 10^{-4}$ colonies/μg/dish for pUH8, and $3.9 \times 10^{-4}$ colonies/μg/dish for pUH9. There were no great differences among the three plasmids.

Then, for the G4I8-resistant colonies obtained by transfection, the 96-well plate culture supernatants were assayed for plasminogen activator (PA) activity by the fibrin-agar plate method. For each plasmid, about I30 clones were examined for PA production. Ten clones in the case of pUH7, 8 clones in the case of pUH8 and I6 clones in the case of pUH9 showed a production of not less than 25 IU/ml. In particular, in the case of pUH9, one clone showed a production as high as I00 IU/ml or more. However, the status of growth and multiplication differed from clone to clone on the 96-well plates and therefore exact comparison with respect to productivity could not be made. Therefore, these clones were cultivated on a large scale and compared with respect to PA production. More specifically, each clone was cultured on Ham's FI2 medium containing I0% (w/v) FCS and when the culture became confluent, the PA production was determined. As a result, transfection with pUH7 gave a clonal strain, U7-2, showing a maximum productivity of about I00 IU/ml/day and transfection with pUH9 gave a clonal strain, U9-25, showing a productivity of about I3 IU/ml/day, while transfection with pUH8 gave a clonal strain, U8-65, showing a productivity of 30 IU/ml/day at most. For each of these clones, the PA activity of the culture supernatant was completely neutralized by anti-urokinase polyclonal anti body. The three strains U7-2, U8-65 and U9-25 highly productive of mutant human PUK were cultured for a period of time and changes in productivity were followed. The strain U9-25 did not show any substantial changes in productivity for about one month. As a result of rescreening of these highly productive strains, there were obtained a clonal strain, U7-2-23, showing a high productivity of 222 IU/ml/day from the strain U7-2, a clonal strain, U8-65-34, showing a productivity of 52 IU/ml/day from the strain U8-65, and a clonal strain, U9-25-6, showing a high productivity of 200 IU/ml/day from the strain U9-25. These strains obtained by rescreening all showed no substantial decrease in productivity even after passage for about one month.

### (7) Culturing method

For the passage of C-68-53 cells and human PUK mutant-producing CHO cells, Ham's FI2 medium containing I0% (w/v) FCS was used. For property analysis and activity assay, the following culturing was carried out. First, the cells were grown on Ham's FI2 medium containing I0% (w/v) FCS until confluency. The cells were then washed well two or three times with Ham's FI2 medium (serum-free) so that serum components could be removed as completely as possible. Thereafter, the cells were maintained in Ham's FI2 medium (serum-free) or the same medium containing I0% (w/v) FCS for 2 to 3 days. In case of protease inhibitor addition, aprotinin was added to a concentration of I0 KIU/ml. After completion of cultivation, the culture supernatant was centrifuged (3,000 rpm, 5 minutes, Hitachi model 05P-2II3 table centrifuge) and stored in a frozen state at -40°C until use thereof.

The experiments on the effects of medium and additives in serum-free cultivation of human PUK mutant-producing CHO cell strains were carried out as follows: Cells were suspended in Ham's FI2 medium, FI2-MW medium (mixture of Ham's FI2 medium and modified Waymouth medium) or RITC medium, each containing 5% (v/v) FCS, and transferred to 75 cm³ flasks ($2 \times 10^6$ cells per flask). After 2 days of incubation, the cells were washed three times with the corresponding serum-free medium and 25 ml of a serum-free, additive-containing culture medium was poured into each flask. Thereafter, the medium was replaced with a fresh portion at 2-day intervals.

## (8) Property analysis of human PUK mutants

Cells were multiplied until confluent. Then, maintenance culturing was conducted using three kinds of medium, namely medium containing I% (w/v) FCS, serum-free medium containing aprotinin, and the culture supernatants from these cultures were subjected to fibrin autography. For all the human PUK mutant-producing strains, the culture supernatant gave a band showing PA activity in the vicinity of molecular weight 45,000. While serum-free culture supernatants gave only a band in the neighborhood of a molecular weight of 45,000, the supernatants obtained from cultures in the presence of I% (v/v) FCS gave a further band indicating a lower molecular weight substance and one more band indicative of higher molecular weight complex although the amounts of these substances were fairly small.

Western blotting was then conducted with the same culture supernatants as mentioned above. Each culture supernatant was subjected to SDS-polyacrylamide gel electrophoresis, followed by Western blotting analysis using antiurokinase polyclonal antibody. The culture supernatants obtained by the use of the strains U7-2, U8-65 and U9-25 gave a main band reactive with the antibody in the neighborhood of a molecular weight of 45,000. Reduction treatment did not cause a shifting of the band to the lower molecular weight side. On the other hand, with C-68-53, i.e., a natural human PUK-producing cell line, the culture supernatant gave a single band corresponding to a molecular weight of about 50,000 under reducing conditions as well as under non-reducing conditions. Urine-derived high-molecular urokinase gave a single band corresponding to a molecular weight of 50,000 under non-reducing conditions and, under reducing conditions, it gave a single band corresponding to a molecular weight of 33,000.

The above results indicate that the three kinds of human PUK mutant as produced in CHO cells were all in the single chain form with a molecular weight of 45,000. The difference of about 5,000 in molecular weight from natural human PUK agreed with the molecular weight value calculated for the number (about 40) of amino acid residues in the EGF domain region deleted therefrom.

The culture supernatants obtained by serum-free culture and culture with I0% (v/v) FCS were assayed for PA activity by the fibrin-agar plate method and for antigen quantity by the RPHA method. Each clone gave no substantial difference between the biological activity value and the activity value calculated from the antigen quantity, irrespective of the presence or absence of serum (Table 9).

Table 9: Production of human PUK mutants by CHO cells

| Cells Cultured | Serum-free medium | | 10% Fetal calf serum-containing medium | |
|---|---|---|---|---|
| | Fibrin plate method (IU/ml) | RPHA method (units/ml) | Fibrin plate method (IU/ml) | RPHA method (units/ml) |
| U7-2 | 90 | 75 | 130 | 90 |
| U8-65 | 8 | 8 | 7 | 8 |
| U9-25 | 150 | 100 | 150 | 150 |
| C-68-53 | 55 | 75 | 45 | 60 |

The above findings demonstrate that the deletion of the EGF domain does not affect the expression of PA activity.

Thereafter, whether the three human PUK mutants produced in CHO cells are proenzymes and whether they are activated by plasmin in the same manner as natural human PUK were investigated (Table l0).

Table 10:  Conversion of human PUK mutants produced in CHO cells to the active form by plasmin treatment

| Cells cultured | PA activity (units/ml) | | Fibrin-agar plate method |
|---|---|---|---|
| | Plasmin (−) | After plasmin treatment (+) | |
| U7-2 | <3.2 | 85 | 90 |
| U8-65 | <3.2 | 16 | 16 · |
| U9-25 | <3.2 | 120 | 150 |
| C-68-53 | <3.2 | 67 | 55 |

For each clone, urokinase activity could not be detected without plasmin treatment. For each clone, plasmin treatment carried out under the same conditions as in the activation of natural human PUK increased the urokinase activity to a level almost equal to the activity value obtained by the fibrin-agar plate method.

It was thus revealed that these human PUK mutants are all produced as proenzymes can be converted satisfacto rily to the active form by plasmin treatment under the same conditions as used in the case of natural human PUK.

(9) MEthod of purification

Chelating Sepharose 6B (l00 ml) was packed into a column, 2.5 $\times$ 20 cm in size, and washed with 500 ml of 0.05 M EDTA-NaOH, pH 8.0, and 500 ml of water in that order. Then, 500 ml of 0.035 M $ZnCl_2$ (pH 4.2) was poured into the column for binding of the $Zn^{2+}$ ion to the gel. Unbound $Zn^{2+}$ ions were washed away with water and the column was equilibrated with a buffer, 0.02 M Tris-HCl, I M NaCl, aprotinin (l0 KIU/ml), 0.0l% (w/v) Tween 80, pH 7.5 (buffer A). The supernatant of a serum-free culture of CHO cells producing a human PUK mutant was then supplied to the column at a flow rate of 200 ml/hr (40 ml/hr/cm²) using a perista-pump. The column thus charged with the sample was washed with buffer A and the human PUK mutant was eluted on a linear imidazole concentration gradient made up with l20 ml of buffer A and l20 ml of buffer A containing 0.05 M imidazole. A similar experiment was carried out using Chelating Sepharose Fast Flow Column (l00 ml, 2.5 $\times$ 20 cm). In this case, however, the rate of supply of the human PUK mutant-producing CHO cell culture supernatant was 400 ml/hr (80 ml/hr/cm²). The active eluate fraction from the Chelating Sepharose 6B column was poured into an anti-UK IgG-Sepharose 4B column (8 ml, l.5 $\times$ 4 cm). This column was washed with 0.5 M NaCl-0.l M sodium phosphate-0.0l% (w/v) Tween 80, pH 7.0, and the human PUK mutant was eluted with 0.5 M NaCl-0.2 M glycine-HCl-0.0l% (w/v) Tween 80, pH 2.5. The active eluate fraction was adjusted to pH 7.0 by addition of solid-form Tris and poured into a p-aminobenzamidine-Sepharose 6B column (20 ml, l.5 $\times$ l0 cm). This column was washed with 0.4 M NaCl-0.l M sodium phosphate-0.0l% (w/v) Tween 80, pH 7.0, and the substance adsorbed was eluted with 0.4 M NaCl-0.l M sodium acetate-0.0l% (w/v) Tween 80, pH 4.0. By this procedure, the human PUK mutant was recovered into an unadsorbed fraction.

In the above manner, a human PUK mutant as produced in a serum-free medium with a specific activity of 27 IU/$A_{280}$ could be purified to a specific activity of 6.9 $\times$ l0⁴ IU/$A_{280}$; the degree of purification was 2,4ll times and the recovery was 7l%.

(l0) Amino acid sequences of human prourokinase mutants

The amino acid sequence in the amino terminal region of each of the human PUK mutants produced by the strains U7-2 (a strain of CHO KI transfected with the plasmid pUH7), U8-65-34 (a strain of CHO KI transfected with the plamid pUH8) and U9-25 (a strain of CHO KI transfected with the plasmid pUH9) was determined by using a gaseous-phase protein sequencer and compared with the amino acid sequence of human kidney cell-derived PUK (Kasai, S. et al., J. Biol. Chem., 260 , 12382 (1985)) (Table II).

Table 11

(I):   Ser-Asn-Glu-Leu-His-Gln-Val-Pro-Ser-Asn-Cys-Asp-Cys-Leu-Asn-Gly-Gly-Thr-Cys-Val-Ser-Asn-Lys-Tyr-Phe-Ser-Asn-Ile-His-Trp-
(II):  Ser-Asn-Glu-Leu-His-Gln-Val-Pro-Ser-
(III): Ser-Asn-Glu-Leu-His-Gln-Val-Pro-Ser-
(IV):  Ser-Asn-Glu-Leu-His-Gln-Val-Pro-Ser-

(I):   Cys-Asn-Cys-Pro-Lys-Lys-Phe-Gly-Gly-Gln-His-Cys-Glu-Ile-Asp-Lys-Ser-Lys-Thr-Cys-Tyr-Glu-Gly-Asn-Gly-His-Phe-Tyr-Arg-Gly- ...
(II):                                      -Glu-Ile-Asp-Lys-Ser-Lys- * - * -Tyr-Glu-Gly- ...
(III):                                             -Lys-Ser-Lys-Thr- * -Tyr-Glu-Gly-Asn-Gly-His- ...
(IV):                                                     - * -Tyr-Glu-Gly-Asn-Gly-His-Phe-Tyr-Arg-Gly- ...

*: The gaseous-phase protein sequencer failed to identify the asterisked amino acid residues.

(I): Human kidney cell-derived PUK

(II): U7-2 cell strain-derived PUK mutant

(III): U8-65-34 cell strain-derived PUK mutant

(IV): U-9-25 cell strain-derived PUK mutant

0 253 241

As a result, it was revealed that the human PUK mutants derived from the strains U7-2, U8-65-34 and U9-25 were deficient in Asn-l0-Cys-42, Asn-l0-Asp-45 and Asn-l0-Thr-49 of human kidney cell-derived PUK, respectively. These human PUK mutants had a molecular weight of about 45,000, which was smaller by about 5,000 than the molecular weight of human kidney cell-derived PUK. This molecular weight difference agreed with the number of the amino acid residues (33 to 40) deficient in the human PUK mutants. Based on the above results, it is estimated that there was no deletion, mutation or the like in other regions than the Asn-l0-Thur-49 region.

(II) Half-life in blood

Comparison between the human PUK mutants obtained in this example and human kidney cell-derived PUK (J. Biol. Chem., 260, 12377 (1985)) was made with respect to half-life in rat blood. More specifically, rats were anesthesized with ether and the carotid artery was cannulated. After administration of the $^{125}$I-labeled form of the human PUK mutant or human kidney cell-derived PUK into the caudal vein at a dose of $10^6$ cpm, rat blood samples were taken at timed intervals via the cannule inserted into the carotid artery and measured for radioactivity contained therein using a gamma counter.

The half-lives in blood of the human PUK mutants obtained in this example were determined in the same manner as in Example I. The results are shown in Table l2 below.

Table l2: Half-life in Blood (min.)

| | |
|---|---|
| Human kidney cell-derived PUK | l.63 ± 0.05 |
| Mutant PUK deficient in the Asn-l0-Cys-42 region | 8.24 ± l.46 |
| Mutant PUK deficient in the Asn-l0-Asp-45 region | 6.25 ± 0.32 |
| Mutant PUK deficient in the Asn-l0-Thr-49 region | 7.l3 ± 0.33 |

The above results show that the EGF domain region-deficient human PUK mutants have longer half-lives in blood as compared with human kidney cell-derived PUK. Similar results were obtained also in rabbits. These results demonstrate that the EGF domain region in the PUK molecule is deeply involved in the kinetics of PUK in the body. Furthermore, the above results demonstrate the substitution of one or more amino acid residues occurring in the EGF domain region with one or more different amino acid residues can expectedly give human PUK mutants deficient in EGF domain function but prolonged in half-life in blood.

EXAMPLES 3 TO 5

Expression vectors for use in Escherichia coli, Bacillus subtilis and yeasts, respectively for the expression of a human PUK mutant deficient in Asn(l0)-Asp(45) were constructed as shwon in Fig. l0 (construction of an expression vector for the expression Asn-l0-Asp-45-deficient human PUK mutant in Escherichia coli), Fig. II (construction of an expression vector for the secretory expression of said human PUK mutant in Bacillus substilis) and Fig. l2 (construction of an expression vector for the expression of said human PUK mutant in yeasts).

The activation with plasmin was carried out by heating at 37°C for 30 minutes at a plasmin concentration of 0.4 CU/ml. To l00 μl of a plasmin-treated or untreated sample, there was added 5 μl of aprotinin (2 × l0⁴ KIU/ml) for inactivation of the plasmin used for activation. Then, 695 μl of buffer A (0.05 M Tris-HCl, 0.038 M NaCl, 0.2% (w/v) BSA, pH 8.8) and l00 μl of an aqueous solution of S-2444 (l.5 mg/ml) were added and changes in absorbance at 405 nm were measured. By comparison with the absorbance values as measured for known concentrations of urine-derived urokinase (Reference Standard GCC, l,l50 IU/ampoule), the urokinase activity of each sample was determined.

As described hereinabove, the present invention, in one aspect thereof, demonstrate that a region (amino acid sequence) determining the half-life in blood (mainly the take-up by hepatocytes) should be present within the region composed of 33 amino acid residues from Asn-l0 to Cys-42.

From the structural viewpoint, this region can be divided into three portions, namely Asn-10 to Cys-19, Val-20 to Asn-32, and Cys-33 to Cys-42. In view of the results of the secondary structure analysis of the Asn-10-Cys-42 region by the method of Chou and Fasman and the report on the binding of the epidermal growth factor (EGF), which is homologous to said region, to its receptor (Blomquist, M.C. et al., Proc. Natl. Acad. Sci. USA, 81, 7363 (1984)), among others, it is considered that the region concerned in the take-up by hepatocytes should be present within the Val-20-Cys-42 portion. The fact that the Cys-33-Cys-42 portion alone is a highly hydrophilic amino acid region among the Asn-10-Cys-42 region and the report describing that the region (Cys-33 to Cys-42) in the amino acid sequence of epidermal growth factor, which is highly homologous to said Cys-33-Cys-42 portion, is associated with the affinity for (binding to) the epidermal growth factor receptor (Nestor, J.J. et al., Biochem. Biophys. Res. Commun. , 129. 226 (1985)), among others, lead to a presumption that the Cys-33-Cys-42 portion of human prourokinase is involved in the take-up by hepatocytes.

Therefore, human PUK mutants in which the entire or part of the Val-20-Cys-42 region of human PUK is absent or is replaced by one or more different amino acid residues, DNA sequences coding for such human PUK mutants, and DNA sequences coding for human PUK mutants in which the entire or part of the Cys-33-Cys-42 region of human PUK is absent or replaced with one or more amino acid residues are also suitable embodiments of the present invention.

In accordance with the present invention, it is possible to provide human PUK mutants of physiological significance as urokinase precursors. Said human PUK mutants have significantly prolonged half-lives as compared with known human PUK species and urokinase. Thus, the invention provides more ideal fibrinolytic enzymes which are expected to produce a beneficial effect in the field of medicine.

While the invention has been described in detail and with reference to specific embodiments thereof, it will be apparent to one skilled in the art that various changes and modifications can be made therein without departing from the spirit and scope thereof.

## Claims

1. A human prourokinase mutant in which the entire or a partial epidermal growth factor domain of human prourokinase is deleted or is replaced by one or more different amino acid residues.

2. A DNA sequence which codes for a human prourokinase mutant in which the entire or a partial epidermal growth factor domain of human prourokinase is deleted or is replaced by one or more different nucleic acid residues.

3. A plasmid having a DNA sequence inserted therein, said DNA sequence coding for a human prourokinase mutant in which the entire or a partial epidermal growth factor domain of human prourokinase is deleted or is replaced by one or more different nucleic acid residues.

4. A host transformed with a plasmid having a DNA sequence inserted therein, said DNA sequence coding for a human prourokinase mutant in which the entire or a partial epidermal growth factor domain of human prourokinase is deleted or is replaced by one or more different nucleic acid residues.

5. The host as claimed in claim 4, wherein the host is an animal cell.

6. A method of producing a human prourokinase mutant in which the entire or a partial epidermal growth factor domain of human prourokinase is deleted or is replaced by one or more different amino acid residues which comprises inserting a DNA sequence and coding for said human prourokinase mutant into a plasmid so as to be expressed in a host transformed with said plasmid.

7. The human prourokinase mutant as claimed in claim 1, wherein the region from asparagine (10) to cysteine (42) or the region from asparagine (10) to aspartic acid (45) or the region from asparagine (10) to threonine (49) is missing.

8. The DNA sequence as claimed in claim 2, wherein the region from asparagine (10) to cysteine (42) or the region from asparagine (10) to aspartic acid (45) or the region from asparagine (10) to threonine (49) is missing.

9. The plasmid as claimed in claim 3, wherein the region from asparagine (10) to cysteine (42) or the region from asparagine (10) to aspartic acid (45) or the region from asparagine (10) to threonine (49) is missing.

10. The host as claimed in claim 4, wherein the region from asparagine (10) to cysteine (42) or the region from asparagine (10) to aspartic acid (45) or the region from asparagine (10) to threonine (49) is missing.

11. The method as claimed in claim 6, wherein the region from asparagine (10) to cysteine (42) or the region from asparagine (10) to aspartic acid (45) or the region from asparagine (10) to threonine (49) is missing.

Claims for the following Contracting State : ES

1. A method of producing a human prourokinase mutant in which the entire or a partial epidermal growth factor domain of human prourokinase is deleted or is replaced by one or more different amino acid residues which comprises inserting a DNA sequence and coding for said human prourokinase mutant into a plasmid so as to be expressed in a host transformed with said plasmid.

2. Method for producing a DNA sequence which codes for a human prourokinase mutant which comprises deleting the entire or a partial epidermal growth factor domain of human prourokinase or replacing it by one or more different nucleic acid residues.

3. Method of producing a plasmid which comprises inserting a DNA sequence coding for a human prourokinase mutant in which the entire or a partial epidermal growth factor domain of human prourokinase is deleted or is replaced by one or more different nucleic acid residues.

4. Method of transforming a host which comprises use of a plasmid having a DNA sequence inserted therein, said DNA sequence coding for a human prourokinase mutant in which the entire or a partial epidermal growth factor domain of human prourokinase is deleted or is replaced by one or more different nucleic acid residues.

5. Method of transforming a host as claimed in claim 4, wherein the host is an animal cell.

6. Method of producing the human prourokinase mutant as claimed in claim 1, wherein the region from asparagine (10) to cysteine (42) or the region from asparagine (10) to aspartic acid (45) or the region from asparagine (10) to threonine (49) is missing.

7. Method of producing the DNA sequence as claimed in claim 2, wherein the region coding for asparagine (10) to cysteine (42) or asparagine (10) to aspartic acid (45) or asparagine (10) to threonine (49) is deleted.

8. Method of producing the plasmid as claimed in claim 3, wherein the region coding for asparagine (10) to cysteine (42) or the region from asparagine (10) to aspartic acid (45) or the region from asparagine (10) to threonine (49) is deleted.

9. Method for transforming the host as claimed in claim 4, wherein the region from asparagine (10) to cysteine (42) or the region from asparagine (10) to aspartic acid (45) or the region from asparagine (10) to threonine (49) is missing.

10. The method as claimed in claim 1, wherein the region from asparagine (10) to cysteine (42) or the region from asparagine (10) to aspartic acid (45) or the region from asparagine (10) to threonine (49) is missing.

# FIG .1

# FIG. 2

# FIG. 3

SJ : Splicing junction
SS : Signal sequence
NC : Non - coding region

# FIG.4

# FIG.5

# FIG.6

FIG.7

| | 5 | 6 | 7 | 8 | 9 | 46 | 47 | 48 | 49 | 50 |
|---|---|---|---|---|---|---|---|---|---|---|
| | HIS | GLN | VAL | PRO | SER | LYS | SER | LYS | THR | CYS |
| | CAT | CAA | GTT | CCA | TCG | AAG | TCA | AAA | ACC | TGC |
| | GTA | GTT | CAA | GGT | AGC | TTC | AGT | TTT | TGG | ACG |

FIG.8

FIG.9(1)

Hind III  Ncol  BamHI  TapI  TaqI  Ncol  EcoRI

SV40 E/P (ori)   EGF domain   UK cDNA

Hind III + TaqI digestion

Hind III   TaqI   TaqI

Hind III — ClaI fragment of pBR 322

ligation

ClaI
EcoRI
Hind III
pUHI
Apr

ClaI + EcoRI digestion

Sfil  ClaI BamHI (Sfil)
Ncol  Ncol
EcoRI  Hind III
puH2
Apr

ligation

BamHI + EcoRI digestion

BamHI   ClaI  Sfil  Ncol  EcoRI

synthetic oligonucleotide

54                                                                    67
ASH GLY HIS PHE TYR ARG GLY LYS ALA SER THR ASP THR MET
5'-CGATAGGCCGG AAT GGC CAC TTT TAC CGC GGA AAG GCT AGC ACT GAC ACC ATG
   TATCCGGCC  TTA CCG GTG AAA ATG GCG CCT TTC CGA TCG TGA CTG TGG TAC
ClaI        Sfil                                                      Hcol
(TaqI)

to FIG.9(2)

0 253 241

# FIG.9(2)

AatII · NarI
XmnI · M13+
BLEUSCRIBE M13⊙ (3,2Kb)
Apʳ · lacZ · EcoRI / BamHI / HindIII

BamHI–EcoRI fragment of Bleuscribe plasmid

from FIG.9(1)

ligation

BamHI ClaI SfiI NcoI EcoRI
pUH3 Apʳ

ClaI + SfiI digestion

ligation

BamHI NcoI EcoRI
pUH4-6 Apʳ

to FIG.9(3)

synthetic oligonucleotide

```
        50              53
        CYS TYR GLU GLY
5'-CG   TGC TAT GAG GGG AA
        ACG ATA CTC CC
```

```
46                              53
LYS SER LYS THR CYS TYR GLU GLY
5'-CG AAG TCA AAA ACC TGC TAT GAG GGG AA
      TTC AGT TTT TGG ACG ATA CTC CC
```

```
43                                      53
GLU ILE ASP LYS SER LYS THR CYS TYR GLU GLY
5'-CG GAA ATC GAT AAG TCA AAA ACC TGC TAT GAG GGG
      CTT TAG CTA TTC AGT TTT TGG ACG ATA CTC CC
      ClaI
```

0 253 241

# FIG. 9(3)

from FIG.9(2)

→ sequencing

BamHI + NcoI
digestion

BmaHI      NcoI

:P ▮▮▮▨▨▨▨ OH

← HindⅢ—BamHI fragment of pSV-G₁—preUK
(400 bp)

HindⅢ    BamHI   BamHI     NcoI

ligation

HindⅢ     BamH1     NcoI

HindⅢ — NcoI
fragment of
pSV-G₁—preUK
(4,2 kb)

ligation

HindⅢ   BamHI

SV40
E/P
pUH7-9

Apʳ

NcoI

KpnI
BamHI

| 5 | 6 | 7 | 8 | 9 | 46 | 47 | 48 | 49 | 50 |
|---|---|---|---|---|----|----|----|----|-----|
| HIS | GLN | VAL | PRO | SER | LYS | SER | LYS | THR | CYS |
| CAT | CAA | GTT | CCA | TCG | AAG | TCA | AAA | ACC | TGC |
| GTA | GTT | CAA | GGT | AGC | TTC | AGT | TTT | TGG | ACG |

Example

0 253 241

FIG .10

FIG.11

Amy: α-amylase region of
B. subtilis natto
Signal : singal peptide

# FIG.12

GAP : yeast glyceraldehyde-3-
phosphate dehydrogenase
p : promoter
t : terminator

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| P,A | EP - A1 - 0 210 279 (SAGAMI CHEMICAL RESEARCH CENTER et al.)<br><br>* Abstract; claims 1,16,21,25 *<br><br>-- | 1-3,6 | C 12 N 15/00<br>C 12 N 9/72<br>C 07 H 21/04<br>C 12 N 5/00 |
| P,A | EP - A1 - 0 200 451 (GENENTECH, INC.)<br><br>* Claims 1-6,22; fig. 1A *<br><br>-- | 1,6 | |
| D,A | EP - A2 - 0 139 447 (THE GREEN CROSS CORPORATION)<br><br>* Page 12 *<br><br>-- | 1 | |
| D,A | EP - A2 - 0 154 272 (THE GREEN CROSS CORPORATION)<br><br>* Claim 1 *<br><br>-- | 1 | |
| D,A | HOPPE-SEYLER'S ZEITSCHRIFT FÜR PHYSIOLOGISCHE CHEMIE, vol. 363, 1982, Berlin, New York<br><br>W.A. GÜNZLER et al. "The Primary Structure of High Molecular Mass Urokinase from Human Urine. The Complete Amino Acid Sequence of the A Chain"<br>pages 1155-1165<br><br>* Totality *<br><br>-- | | **TECHNICAL FIELDS SEARCHED (Int. Cl.4)**<br><br>C 12 N<br>C 07 H |

The present search report has been drawn up for all claims

| Place of search | Date of completion of the search | Examiner |
|---|---|---|
| VIENNA | 28-09-1987 | WOLF |

| DOCUMENTS CONSIDERED TO BE RELEVANT | | | EP 87109628.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | NUCLEIC ACIDS RESEARCH, vol. 13, no. 8, April 25, 1985, Oxford, Washington, DC<br><br>A. RICCIO et al. "The human uro-kinase-plasminogen activator gene and its promoter"<br>pages 2759-2771<br><br>    * Totality *<br><br>           ----- | | |

**TECHNICAL FIELDS SEARCHED (Int. Cl.4)**

The present search report has been drawn up for all claims

| Place of search | Date of completion of the search | Examiner |
|---|---|---|
| VIENNA | 28-09-1987 | WOLF |